(19) **Europäisches Patentamt / European Patent Office / Office européen des brevets**

(11) **EP 3 715 463 B1**

(12) **EUROPEAN PATENT SPECIFICATION**

(45) Date of publication and mention
of the grant of the patent:
**25.01.2023 Bulletin 2023/04**

(21) Application number: **20164909.2**

(22) Date of filing: **23.03.2020**

(51) International Patent Classification (IPC):
**C12N 15/82** (2006.01)    **C12N 9/88** (2006.01)
**B01J 19/00** (2006.01)    **C07K 19/00** (2006.01)

(52) Cooperative Patent Classification (CPC):
**C12N 15/8257; C12N 9/88; C12Y 402/01001;**
C07K 2319/02; C07K 2319/04; C07K 2319/20;
C07K 2319/95

(54) **METHOD OF MASS-PRODUCING CARBONIC ANHYDRASE IN PLANTS**

VERFAHREN ZUR MASSENPRODUKTION VON KOHLENSÄUREANHYDRASE IN PFLANZEN

PROCÉDÉ DE PRODUCTION EN MASSE D'ANHYDRASE CARBONIQUE DANS LES PLANTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.03.2019 KR 20190032749**

(43) Date of publication of application:
**30.09.2020 Bulletin 2020/40**

(73) Proprietor: **Biocompanion Inc.**
**Pohang-si 37673 (KR)**

(72) Inventors:
- **HWANG, In Hwan**
  **37673 Pohang-si (KR)**
- **RAZZAKMD, Abdur**
  **37673 Pohang-si, (KR)**
- **RYU, Bong Ryul**
  **52710 Jinju-si (KR)**
- **LEE, Dong Wook**
  **37666 Pohang-si (KR)**
- **LEE, Jun Ho**
  **37835 Pohang-si (KR)**
- **KUMARI, Madhu**
  **16042 Uiwang-si (RO)**

(74) Representative: **Dehns**
**St. Bride's House**
**10 Salisbury Square**
**London EC4Y 8JD (GB)**

(56) References cited:
**WO-A2-2008/095057**

- **MD REYAZUL ISLAM ET AL: "Cost-effective production of tag-less recombinant protein in Nicotiana benthamiana", PLANT BIOTECHNOLOGY JOURNAL, vol. 17, no. 6, 8 December 2018 (2018-12-08), pages 1094-1105, XP055683185, GB ISSN: 1467-7644, DOI: 10.1111/pbi.13040**
- **LUCA VIVIANA DE ET AL: "An [alpha]-carbonic anhydrase from the thermophilic bacteriumSulphurihydrogenibium azorenseis the fastest enzyme known for the CO2hydration reaction", BIOORGANIC & MEDICINAL CHEMISTRY, ELSEVIER, NL, vol. 21, no. 6, 29 September 2012 (2012-09-29), pages 1465-1469, XP028987245, ISSN: 0968-0896, DOI: 10.1016/J.BMC.2012.09.047**

Remarks:
The complete document including Reference Table(s) and the Sequence Listing(s) can be downloaded from the EPO website

**Description**

BACKGROUND

**1. Field of the Invention**

The present disclosure relates to a recombinant vector for production of a carbonic anhydrase in a plant and a transformant transformed with the samw.

**2. Discussion of Related Art**

Carbonic anhydrases hydrate carbon dioxide in water or carry out dehydration from bicarbonate ions. Carbonic anhydrase is called a metalloenzyme since it contains a zinc metal ion in an active site thereof. Carbonic anhydrase can be used to specifically collect $CO_2$. When used together with MDEA, carbonic anhydrase exhibits higher $CO_2$-collecting activity than when MDEA is used alone. In addition, carbonic anhydrase is effective in recovering $CO_2$ collected in a MEDA solution. Carbonic anhydrase can recover only $CO_2$ by purely collecting $CO_2$ from a gas mixture containing $CO_2$. Currently, a technique for collecting $CO_2$ from an exhaust gas using methyl diethanol-amine (MDEA), which is an amine-based compound, or a membrane such as Ni-MOF-74, or a technique for purely purifying only $CO_2$ from a gas mixture containing $CO_2$ by a pressurization swinging adsorption (PSA) method is being developed. For use in collecting $CO_2$, it is required to mass-produce carbonic anhydrase at low cost and use the same after purification. In the case of using an MEDA solution including carbonic anhydrase, *E. coli* was used to produce DvCA carbonic anhydrase (CAH derived from *Desulfovibrio vulgaris*) and *E. coil* including carbonic anhydrase was used as it is without separation and purification.

The remarkable development of molecular biology and genetic engineering technology has also been applied to the plant field, and efforts to produce useful physiologically active substances from plants have continued. In the case of producing useful substances in plants, production costs can be dramatically reduced, and various contaminants such as viruses, cancer genes, and enterotoxins, which may be generated as a result of using a method of synthesizing proteins in conventional animal cells or microorganisms and separating and purifying the same, can be fundamentally excluded, and even in a commercialization process, long-term storage and management thereof as seeds are possible unlike animal cells or microorganisms. In addition, when demand for the corresponding useful substance rapidly increases, it is absolutely advantageous in terms of equipment technology or costs required for mass production unlike existing animal cell systems, and thus the useful substance can be supplied in accordance with the increased demand within the shortest period of time.

However, in spite of these merits, in producing proteins in plant cells, a relatively low level of protein expression and difficulties in separation and purification are the biggest drawbacks, compared to other hosts including animal cells. Thus, many studies have been conducted, and there have been attempts to increase protein expression in plant cells and efficiently separate and purify proteins in various ways. In the case of using a plant, there are a method of producing a protein using a transformant, a method of excessively inducing protein expression in a plant using Agrobacterium-mediated transformation and producing a protein therethrough, and the like. For example, Korean Patent Publication No. 10-2018-0084680 discloses a recombinant vector for expressing a target protein in a plant cell, and Korean Patent Publication No. 10-2017-0131207 discloses a method of highly expressing a target protein from a plant by using a RbcS fusion protein, and a method of preparing a composition for oral administration of a medical protein using a target protein-expressing plant. In the case of using a transformant, a method of producing a protein in specific tissue such as leaves or seeds using the whole plant or a method of producing a protein by inducing a cell line from the transformant is used. Although the method through Agrobacterium-mediated transformation is rapid and provides a high expression level, a method including a process of culturing Agrobacterium and infiltrating the culture solution via vacuum is more complicated than the case of using a transgenic plant.

WO2008/095057 discloses a heat-stable carbonic anhydrase and its use for extracting carbon dioxide from a medium.

Islam et al., 2019 Plant Biotechnology Journal 17: 1094-1105 discloses a method for the production of recombinant proteins in plants based on the target gene hII,6.

As a result of having made intensive efforts to facilitate the separation and purification of a carbonic anhydrase in a plant, it was confirmed that, when CBM3, SUMO, and M domains are fused to GcCAHα3 or SazCA, which is a carbonic anhydrase, the production of carbonic anhydrase in a plant is easy, and separation and purification thereof were possible using the same, and a carbonic anhydrase immobilized on the surface of cellulose beads was used in the hydration reaction of carbon dioxide to confirm the finding, thus completing the present disclosure.

SUMMARY OF THE INVENTION

The present invention provides a recombinant vector for producing a carbonic anhydrase in a plant, and a transformant transformed with the recombinant vector, as defined in the appended claims.

According to an aspect of the present disclosure, there is provided a recombinant vector for producing a carbonic anhydrase in a plant, including: (i) a small ubiquitin-related modifier (SUMO); (ii) a carbonic anhydrase; (iii) cellulose-binding module 3 (CBM3); and (iv) M domains located before each of the N-terminal of the SUMO and the N-terminal of the CBM3, respectively,
wherein the gene sequence of each M domain comprises a nucleotide sequence encoding an amino acid sequence of SEQ ID NO: 5, or comprises a nucleotide sequence of SEQ ID NO: 6.

According to an exemplary embodiment of the present disclosure, the carbonic anhydrase may be derived from *Sulfurihydrogenibium azorense* or *Gracilariopsis chorda.*

According to another exemplary embodiment of the present disclosure, a gene sequence of the carbonic anhydrase derived from *Gracilariopsis chorda* may include a nucleotide sequence encoding an amino acid sequence of SEQ ID NO: 1, and a gene sequence of the carbonic anhydrase derived from *Sulfurihydrogenibium azorense* may include a nucleotide sequence encoding an amino acid sequence of SEQ ID NO: 11.

According to another exemplary embodiment of the present disclosure, a gene sequence of the carbonic anhydrase derived from *Gracilariopsis chorda* may include a nucleotide sequence of SEQ ID NO: 2, and a gene sequence of the carbonic anhydrase derived from *Sulfurihydrogenibium azorense* may include a nucleotide sequence of SEQ ID NO: 12.

According to another exemplary embodiment of the present disclosure, a gene of the SUMO may include a nucleotide sequence of SEQ ID NO: 7.

According to another exemplary embodiment of the present disclosure, a gene of the cellulose-binding module 3 (CBM3) may include a nucleotide sequence of SEQ ID NO: 8.

In the present disclosure, the recombinant vector may further include any one promoter selected from the group consisting of a 35S promoter derived from a cauliflower mosaic virus, a 19S RNA promoter derived from a cauliflower mosaic virus, an actin protein promoter of a plant, and an ubiquitin protein promoter.

According to another aspect of the present disclosure, there is provided a transformant transformed with the recombinant vector.

According to an exemplary embodiment of the present disclosure, the transformant may be Agrobacterium.

BRIEF DESCRIPTION OF THE DRAWINGS

The above and other objects, features and advantages of the present disclosure will become more apparent to those of ordinary skill in the art by describing in detail exemplary embodiments thereof with reference to the accompanying drawings, in which:

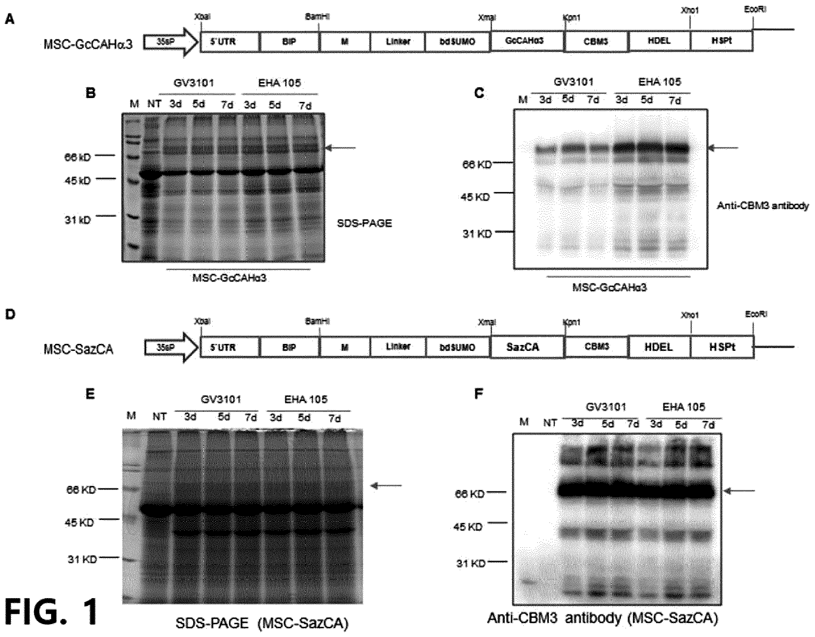

FIG. 1 illustrates the results of confirming the structures and expression levels of MSC-GcCAHα3 and MSC-SazCA, wherein FIG. 1A is a cleavage map of MSC-GcCAHα3, FIG. 1B illustrates results showing the expression level thereof in *N. benthamiana* after staining, with Coomassie blue, a membrane on which western blotting was performed, FIG. 1C illustrates results obtained by reacting MSC-GcCAHα3 with an anti-CBM3 antibody, FIG. 1D is a cleavage map of MSC-SazCA, FIG. 1E illustrates results showing the expression level thereof in *N. benthamiana* after staining, with Coomassie blue, a membrane on which western blotting was performed, and FIG. 1F illustrates results obtained by reacting MSC-SazCA with an anti-CBM3 antibody;

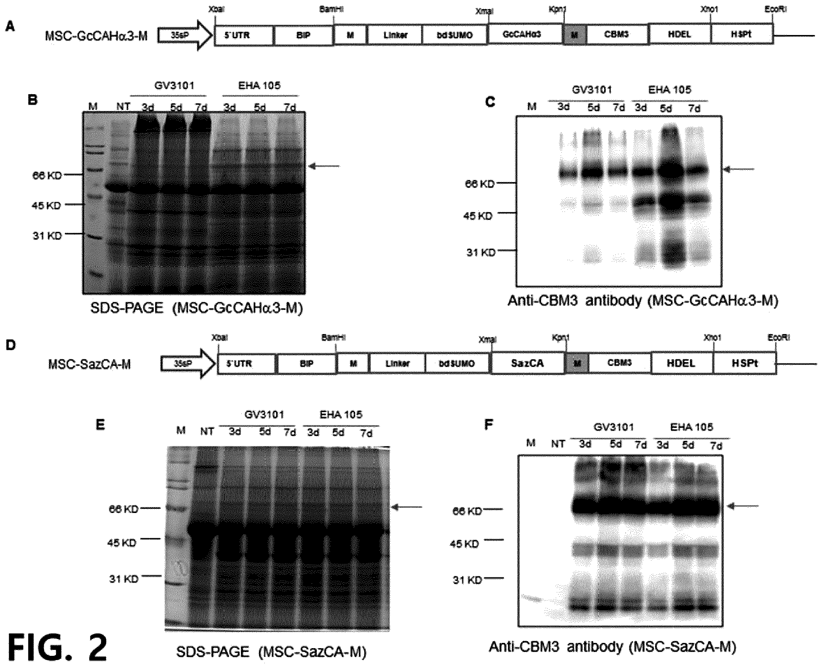

FIG. 2 illustrates the results of confirming the structures and expression levels of MSC-GcCAHα3-M and MSC-SazCA-M, wherein FIG. 2A is a cleavage map of MSC-GcCAHα3-M, FIG. 2B illustrates results showing the expression level thereof in *N. benthamiana* after staining, with Coomassie blue, a membrane on which western blotting was performed, FIG. 2C illustrates results obtained by reacting MSC-GcCAHα3-M with an anti-CBM3 antibody, FIG. 2D is a cleavage map of MSC-SazCA-M, FIG. 2E illustrates results showing the expression level thereof in *N. benthamiana* after staining, with Coomassie blue, a membrane on which western blotting was performed, and FIG. 2F illustrates results obtained by reacting MSC-SazCA-M with an anti-CBM3 antibody;

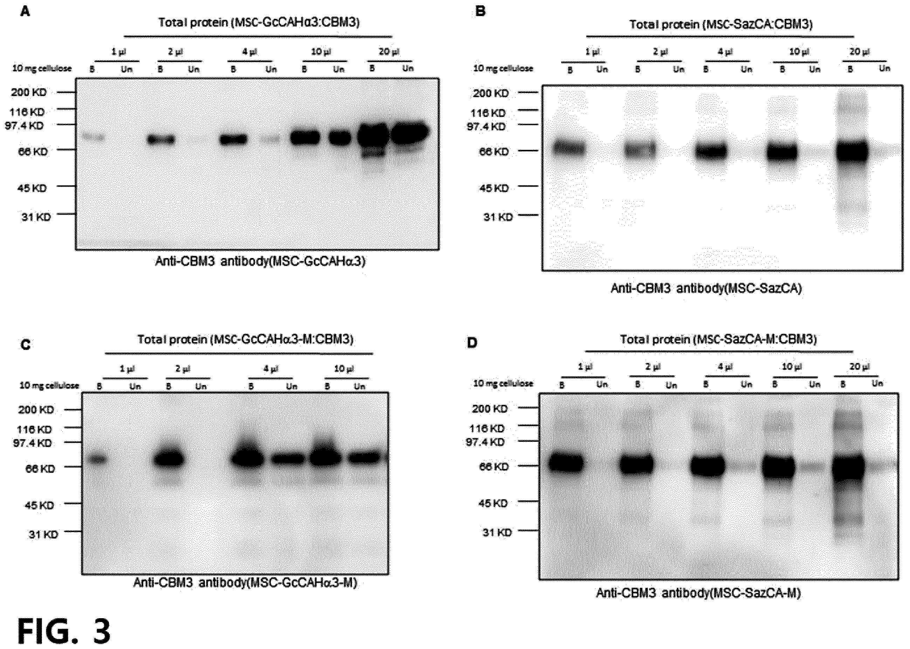

FIG. 3 illustrates the results of confirming the amounts of binding of recombinant proteins MSC-GcCAHa3 (3A), MSC-SazCA (3B), MSC-GcCAHa3-M (3C), and MSC-SazCA-M (3D) to cellulose beads;

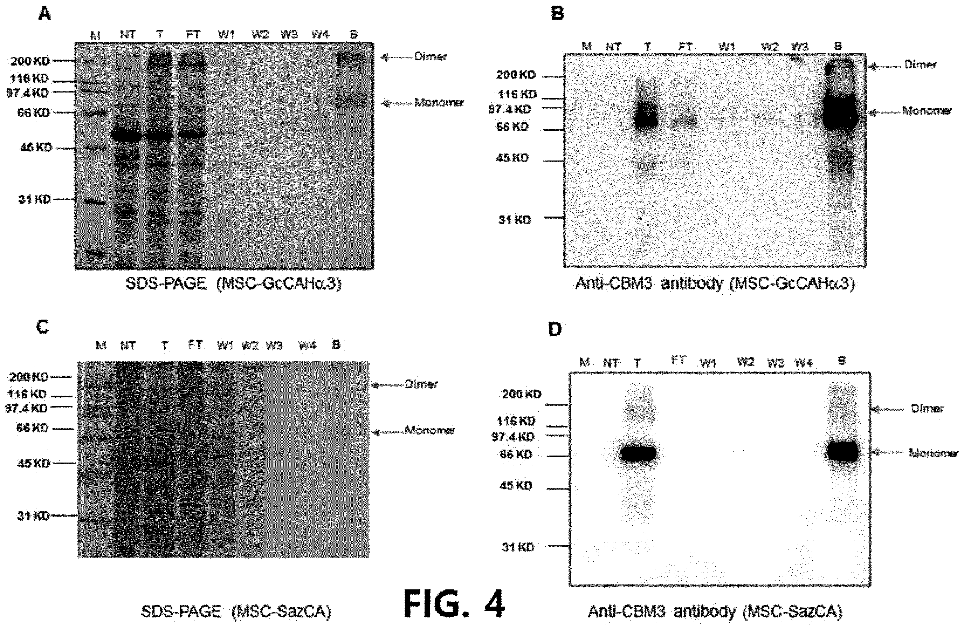

FIG. 4 illustrates the results of purely separating recombinant proteins MSC-GcCAHα3 and MSC-SazCA from a *N. benthamiana* leaf extract by using cellulose beads, wherein FIG. 4A illustrates Coomassie blue staining results obtained after western blotting on a membrane for a protein obtained by boiling and eluting MSC-GcCAHα3, FIG. 4B illustrates results obtained by reacting the membrane with an anti-CBM3 antibody, FIG. 4 illustrates Coomassie blue staining results obtained after western blotting on a membrane for a protein obtained by boiling and eluting MSC-SazCA, and FIG. 4D illustrates results obtained by reacting the membrane with an anti-CBM3 antibody (Lane M, protein size marker; NT, non-transformed wild type *N. benthamiana* protein extract; T, total protein extract of total transformed *N. benthamiana* leaves; FT, flow-through fraction of column; W, washing-off fraction; E, protein recovered from cellulose beads via boiling);

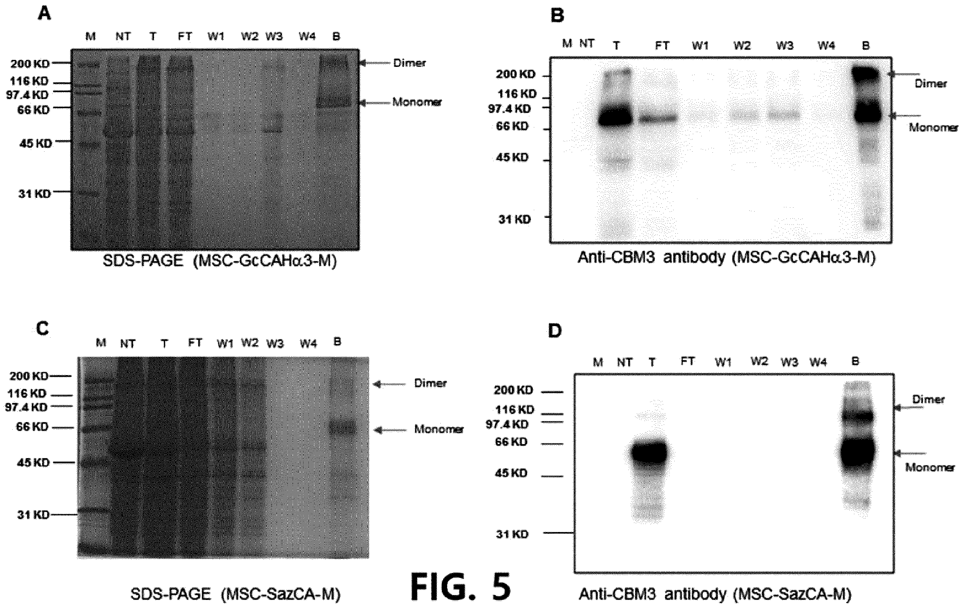

FIG. 5 illustrates results obtained by purely separating recombinant proteins MSC-GcCAHα3-M and MSC-SazCA-M from an *N. benthamiana* leaf extract by using cellulose beads, wherein FIG. 5A illustrates Coomassie blue staining results obtained after western blotting on a membrane for a protein obtained by boiling and eluting MSC-GcCAHα3-M, FIG. 5B illustrates results obtained by reacting the membrane with an anti-CBM3 antibody, FIG. 5C illustrates Coomassie blue staining results obtained after western blotting on a membrane for a protein obtained by boiling and eluting MSC-SazCA-M, and FIG. 5D illustrates results obtained by reacting the membrane with an anti-CBM3 antibody (Lane M, protein size marker; NT, non-transformed wild type *N. benthamiana* protein extract; T, total protein extract of total transformed *N. benthamiana* leaves; FT, flow-through fraction of column; W, washing-off fraction; E, protein recovered from cellulose beads via boiling);

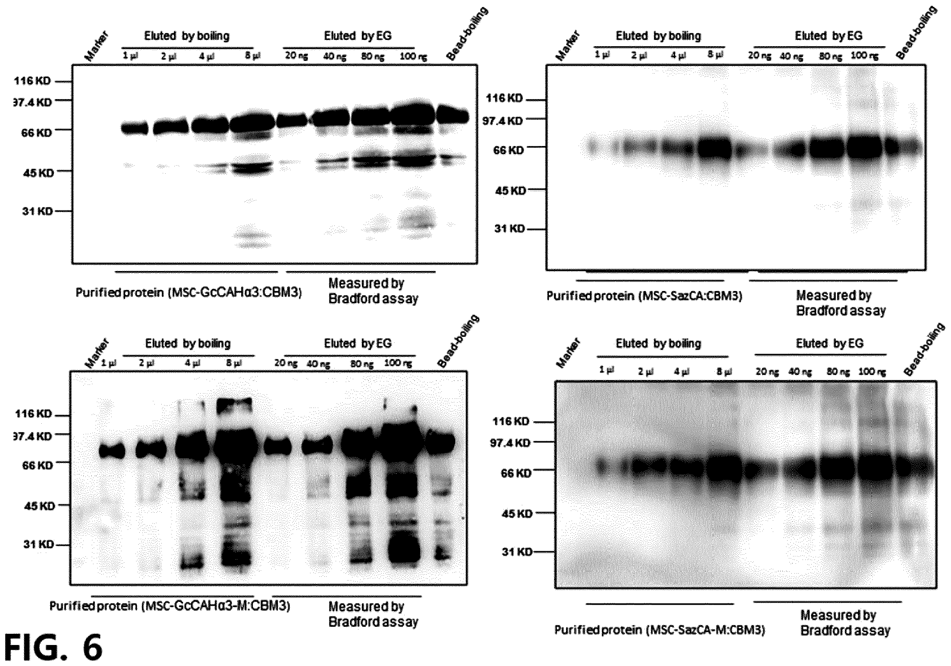

FIG. 6 illustrates the results of confirming the expression levels of recombinant proteins MSC-GcCAHα3 (6A), MSC-SazCA (6B), MSC-GcCAHa3-M (6C), and MSC-SazCA-M (6D) (MSC-GcCAHα3 obtained by boiling was loaded from 1 $\mu$l to 8 $\mu$l (lane 2, 1 $\mu$l; 3, 2 $\mu$l; 4, 4 $\mu$l; 5, 8 $\mu$l) for comparison, a protein eluted with EG was loaded from 20 ng to 100 ng (Lane 6, 20 ng; 7, 40 ng; 8, 80 ng; 9, 100 ng) to be used as a reference for quantitatively comparing the amounts of proteins eluted by boiling, and the proteins were separated by SDS-PAGE, followed by western blot analysis using an anti-CBM3 antibody);

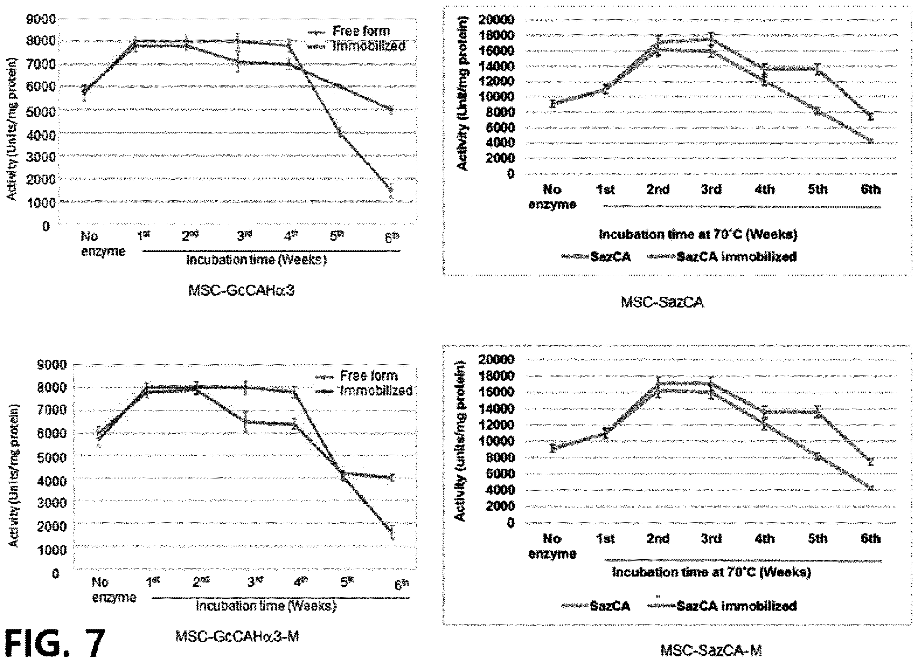

FIG. 7 illustrates the results of comparing the thermal stabilities in the $CO_2$ hydration reaction of carbonic anhydrases produced by recombinant proteins MSC-GcCAHα3 (7A), MSC-SazCA (7B), MSC-GcCAHα3-M (7C), and MSC-SazCA-M (7D);

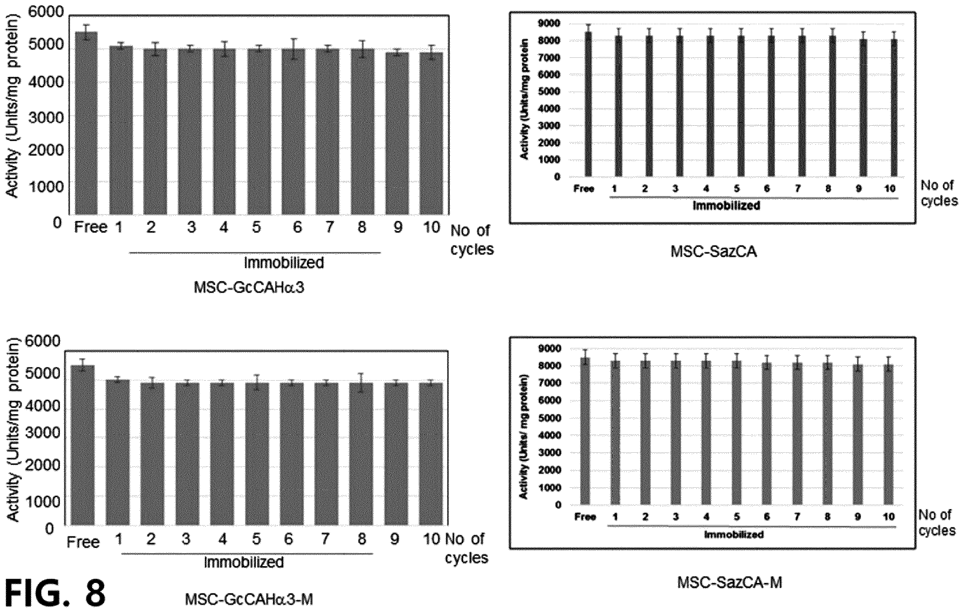

FIG. 8 illustrates the results of confirming the durability in the $CO_2$ hydration reaction of carbonic anhydrases produced by recombinant proteins MSC-GcCAHα3 (8A), MSC-SazCA (8B), MSC-GcCAHα3-M (8C), and MSC-SazCA-M (8D);

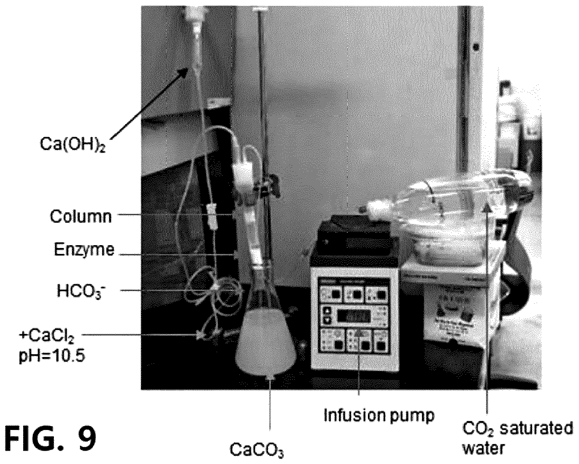

FIG. 9 illustrates the experimental design results of confirming consistency in the $CO_2$ hydration reaction of carbonic anhydrases produced by recombinant vectors MSC-GcCAHα3, MSC-SazCA, MSC-GcCAHα3-M, and MSC-SazCA-M; and

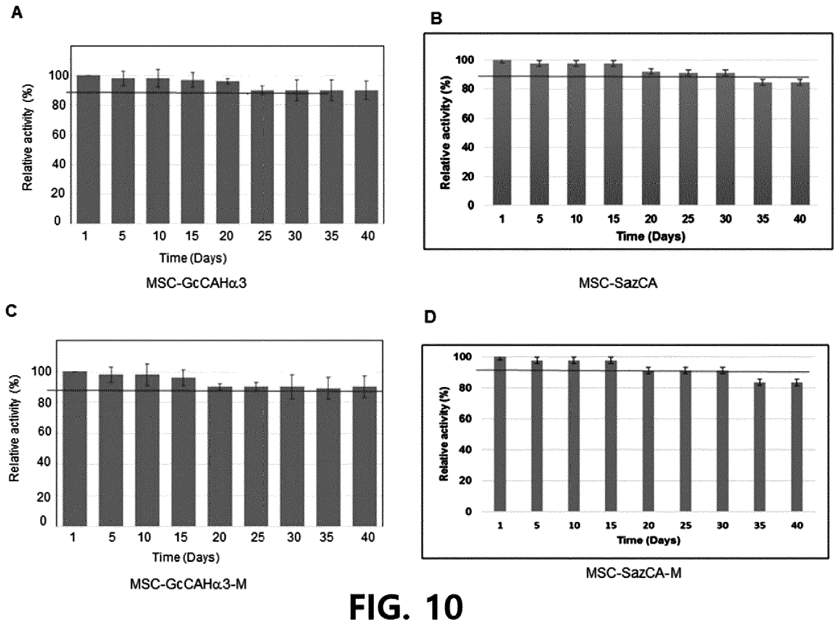

FIG. 10 illustrates the results of confirming the consistency in the $CO_2$ hydration reaction of carbonic anhydrases produced by recombinant vectors MSC-GcCAHα3, MSC-SazCA, MSC-GcCAHα3-M, and MSC-SazCA-M.

## DETAILED DESCRIPTION OF EXEMPLARY EMBODIMENTS

Exemplary embodiments of the present disclosure will be described in detail below with reference to the accompanying drawings. As described above, producing proteins using animal cells or microorganisms causes problems such as various contaminants, e.g., viruses, cancer genes, and enterotoxins, and has limitations in that it is time-consuming and expensive in the case of mass production. To overcome this, a method of producing proteins in a plant has been developed, but this also causes low protein expression and has difficulty in protein separation and purification.

Therefore, the inventors of the present disclosure have developed a method for increasing protein expression in a plant and efficiently separating and purifying the proteins and constructed recombinant vectors. A recombinant vector of the present disclosure includes a carbonic anhydrase, and as a result of having cultured the recombinant vector including a carbonic anhydrase, infiltrated the culture solution into a plant through a syringe infiltration method, mass-produced the same, and purified the same via cellulose beads, a high purification rate was exhibited. In addition, a carbonic anhydrase prepared by the recombinant vector exhibited high thermal stability, could be used for a long period of time due to a high reuse rate thereof, and exhibited a high degree of collecting carbon dioxide.

Hereinafter, embodiments of the present disclosure will be described in detail with reference to the accompanying drawings in such a way that the present disclosure can be carried out by one of ordinary skill in the art without undue difficulty. The present disclosure may be embodied in many different forms and embodiments as set forth herein are not intended to limit the present disclosure.

The present disclosure may provide a recombinant vector for producing a carbonic anhydrase in a plant as defined in the appended claims.

The term "carbonic anhydrase (CA)" as used herein refers to an enzyme that catalyzes the hydration reaction of carbon dioxide as a metal enzyme containing zinc therein. The carbonic anhydrase may be derived from *Sulfurihydrogenibium azorense* or *Gracilariopsis chorda.*

A gene sequence of the carbonic anhydrase derived from *Gracilariopsis chorda,* according to the present disclosure may include a nucleotide sequence encoding an amino acid sequence of SEQ ID NO: 1, and a gene sequence of the carbonic anhydrase derived from *Sulfurihydrogenibium azorense* may include a nucleotide sequence encoding an amino acid sequence of SEQ ID NO: 11. The gene sequence of the carbonic anhydrase derived from *Gracilariopsis chorda* may include a nucleotide sequence of SEQ ID NO: 2, and the gene sequence of the carbonic anhydrase derived from *Sulfurihydrogenibium azorense* may include a nucleotide sequence of SEQ ID NO: 12. Particularly, the gene may include a nucleotide sequence having at least 70%, more preferably at least 80%, even more preferably at least 90%, or most preferably at least 95% sequence homology to the nucleotide sequence of SEQ ID NO: 2 or SEQ ID NO: 12. The "% sequence homology" for a polynucleotide is identified by comparing comparative regions between two optimally aligned sequences, and part of the polynucleotide sequence within the comparative regions may include an addition or a deletion (i.e., a gap) compared to the reference sequence (without any addition or deletion) for the optimum arrangement of the two sequences.

A recombinant vector for producing a carbonic anhydrase in a plant, including an M domain that increases protein expression, a small ubiquitin-related modifier (SUMO) that increases the water solubility of a protein, and cellulose-binding module 3 (CBM3) capable of separating the protein by binding to cellulose beads was constructed. The inventors of the present disclosure linked one M-domain to the N-terminal of SUMO or further fused an additional M domain to the N-terminal of CBM3. Agrobacterium was transformed therewith, and then cultured, followed by infiltration into *Nicotiana benthamiana* using a needleless syringe and a vacuum infiltration method, and a protein was extracted from the *Nicotiana benthamiana.* As a result, it was confirmed that the expression of a carbonic anhydrase was increased in the vector in which two M domains were fused, compared to the vector in which a single M domain was fused (see FIGS. 1 and 2). The inventors of the present disclosure determined the binding capacity of CBM3 to 10 mg of cellulose beads using various amounts of (1 µl to 20 µl) of a total *N. benthamiana* leaf extract. As a result, when 4 µl of the total extract was mixed with cellulose beads, a trace amount of a CBM3 band began to be shown in an unbound region in the case of MSC-GcCAHα3, and at 10 µl and 20 µl, about a half amount of the CBM3 band was shown in the unbound region. From these results, the amount of CBM3 in 4 µl of the total protein extract was determined as an amount that slightly exceeded saturation of the cellulose beads. When up to 2 µl of the total extract was mixed with cellulose beads, the CBM3 band was not shown in an unbound region in the case of MSC-GcCAHα3-M, and at 4 µl and 10 µl, about a half amount of CBM3 was shown in the unbound region (see FIG. 3). When up to 20 µl of the total protein extract was mixed with cellulose beads, the CMB3 band was not shown in an unbound region in the case of MSC-Saz and MSC-Saz-M. It was confirmed that MSC-GcCAHα3, MSC-Saz, MSC-GcCAHα3-M, and MSC-Saz-M tightly bound to cellulose beads and were nearly purely separated and purified. According to western blot results and Coomassie blue staining results, MSC-GcCAHα3, MSC-Saz, MSC-GcCAHα3-M, and MSC-Saz-M were shown at the 68 kD position, and were also shown as other bands with a larger size than that at the 116 kD position (see FIGS. 4 and 5). The protein at the 68 kD position is also present as a doublet, which is predicted by differential glycosylation of N-glycosylation sites present in an M domain and the like. In addition, bands above the 116 kD position were determined to be multimers. As a result of having separated and purifying the total *N. benthamiana* leaf extract using cellulose beads and measuring the amounts of proteins thereof, a protein expression level of 100 µg/g fresh weight was confirmed (see FIG. 6). As a result of having conducted a thermal stability experiment while GcCAHα separated and purified from the recombinant MSC-GcCAHα3, MSC-Saz, MSC-GcCAHα3-M, and MSC-Saz-M, and GcCAHα3 separated and purified from *E. coli* were continuously cultured at 70 °C, GcCAHα immobilized to cellulose beads exhibited higher thermal stability than that of GcCAHα3 separated and purified from *E. coli* (FIG. 7). The degree of reuse of the activity of GcCAHα3 separated and purified from MSC-GcCAHα3, MSC-Saz, MSC-GcCAHα3-M, and MSC-Saz-M was measured in such a way that 10 µg of the protein was added to $CO_2$-saturated water (4 mL) and mixed with 6 mL of 20 mM Tris buffer (pH 8.3) cooled with ice to induce reaction, the time taken for the pH to change from 8.3 to 6.3 was measured to measure the activity of the protein. The specific activity of free-form GcCAHα3 was 5722 Wilber-Anderson units (WAU)/mg, and the specific activity of GcCAHα3 separated and purified from MSC-GcCAHα3, MSC-Saz, MSC-GcCAHα3-M, and MSC-Saz-MD, and immobilized to cellulose beads was 5200 WAU/mg (see FIG. 8). This is because MSC-GcCAHα3 separated and purified from a plant has a molecular weight that is twice the molecular weight of a free form produced from *E. coli.* Thus, MSC-GcCAHα3 produced in a plant has an effect of having almost two-fold actual activity. After the reaction, cellulose beads having the protein bound thereto were recovered and a new identical reaction was induced, and even after repeating the reaction 10 times, there was completely no change in activity. Through this, MSC-GcCAHα3 immobilized onto cellulose beads has an effect of being used for a long period of time. In addition, through an experimental design as shown in FIG. 9, carbon dioxide of GcCAHα3 separated and purified from MSC-GcCAHα3, MSC-Saz, MSC-GcCAHα3-M, and MSC-Saz-MD, and immobilized onto cellulose was hydrated in water or dehydration from bicarbonate ions was carried out, and as a result, the activity was slightly decreased over time, but 90% or more thereof was maintained for 40 days (see FIG. 10).

The M domains of the present disclosure are fused to increase the expression of a protein, and the gene sequence of the M domains may be located after the C-terminal of the carbonic anhydrase, and the gene sequence of the M domains may include a nucleotide sequence encoding an amino acid sequence of SEQ ID NO: 5 or SEQ ID NO:.

The "% sequence homology" for a polynucleotide is identified by comparing comparative regions between two optimally aligned sequences, and part of the polynucleotide sequence within the comparative regions may include an addition or a deletion (i.e., a gap) compared to the reference sequence (without any addition or deletion) for the optimum arrangement of the two sequences.

The small ubiquitin-related modifier (SUMO) of the present disclosure is covalently linked through the C-terminal to ε-amino residues of lysine present in various target proteins, thereby forming isopeptides. To fuse the SUMO to a target protein, a SUMO-specific protease must remove the extra site present at the C-terminal site from a SUMO precursor. In this regard, a SUMO-specific protease recognizes a glycine-glycine motif that is specifically present at the C-terminal of the SUMO domain, thereby removing the extra site present at the C-terminal of a GG sequence. When producing SUMO from a SUMO precursor, a SUMO protease recognizes the SUMO domain and cleavages the C-terminal regions of two glycines, and thus can exhibit extremely high specificity. In addition, a SUMO protease barely exhibits specificity for the type of amino acids following two glycine residues, and thus various proteins may be attached after di-glycine residues, and a mature SUMO domain with di-glycine at the C-terminal thereof is accurately removed therefrom so that the SUMO protease has the property that enables a specific protein not to have extra amino acids. The SUMO of the present disclosure may include a nucleotide sequence of SEQ ID NO: 7, and specifically, may include a nucleotide sequence having at least 70%, more preferably at least 80%, even more preferably at least 90%, and most preferably at least 95% sequence homology to the nucleotide sequence represented by SEQ ID NO: 7. The "% sequence homology" for a polynucleotide is identified by comparing comparative regions between two optimally aligned sequences, and part of the polynucleotide sequence within the comparative regions may include an addition or a deletion (i.e., a gap) compared to the reference sequence (without any addition or deletion) for the optimum arrangement of the two sequences.

Cellulose-binding module 3 (CBM3) of the present disclosure is a cellulose-binding domain obtained from *C. thermocellum,* which shows a very high capacity of binding to cellulose, and thus has several properties for use as an affinity tag for pure protein separation. The CBM3 is specific to amorphous cellulose and has a high degree of binding, and when a target protein is fused to CBM3, the CBM3 interferes less with the fused target protein, and also helps the folding of the fused target protein. In microorganisms, CBM3 has been reported to increase protein production yield. The cellulose to which the CBM3 binds is environmentally friendly as an affinity resin, and has various merits such as very low costs, and the like. Cellulose has little chemical reactivity, so buffer solutions of various compositions can be used, and it is known that cellulose will not react with proteins. In addition, various forms of cellulose may be commercially available. Therefore, cellulose has various conditions suitable for use as an affinity resin most suitable for pure protein separation. In practice, due to human friendliness thereof, cellulose has already been used in a variety of medical products and products applicable to the human body. The CBM3 of the present disclosure may include a nucleotide sequence of SEQ ID NO: 8, and specifically, may include a nucleotide sequence having at least 70%, more preferably at least 80%, even more preferably at least 90%, and most preferably at least 95% sequence homology to the nucleotide sequence represented by SEQ ID NO: 8. The "% sequence homology" for a polynucleotide is identified by comparing comparative regions between two optimally aligned sequences, and part of the polynucleotide sequence within the comparative regions may include an addition or a deletion (i.e., a gap) compared to the reference sequence (without any addition or deletion) for the optimum arrangement of the two sequences.

A gene of the recombinant protein includes the signal sequence of BiP and HDEL, which is an ER retention signal, respectively at the N-terminal and C-terminal thereof, and thus may have an effect of inducing accumulation at a high concentration in the endoplasmic reticulum (ER). The recombinant vector may further include any one selected from the group consisting of a chaperone binding protein (BiP) and a his-asp-glu-leu (HDEL) peptide, and the BiP may include a nucleotide sequence of SEQ ID NO: 4, and the HDEL may include a nucleotide sequence of SEQ ID NO: 9.

In addition, the recombinant vector may further include a translation amplification sequence 5'-UTR, and the 5'-UTR may include a nucleotide sequence of SEQ ID NO: 3.

The term "recombinant" is intended to mean a cell that replicates heterogeneous nucleic acids, or expresses the nucleic acids or expresses a peptide, a heterogeneous peptide, or a protein encoded by a heterogeneous nucleotide. Recombinant cells can express a gene or a gene fragment, which is not found in natural forms of the cells, in one form selected from sense and antisense forms. In addition, a recombinant cell can express a gene that is found naturally, but the gene is modified and re-introduced into the cell by artificial means.

The term "recombinant expression vector" refers to a bacterial plasmid, a phage, a yeast plasmid, a plant cell virus, a mammalian cell virus, or other vectors. Generally, any plasmid and vector may be used as long as they are capable of replicating and being stabilized in a host. An important characteristic of the expression vector is that the vector has an origin of replication, a promoter, a marker gene, and a translation control element. The recombinant expression vector and an expression vector including suitable transcriptional/translational control signals may be constructed by methods well known to those of ordinary skill in the art. Such methods include *in vitro* recombinant DNA techniques, DNA synthesis techniques, *in vivo* recombinant techniques, and the like. The DNA sequence can be effectively linked to a suitable promoter in the expression vector to drive mRNA synthesis.

An exemplary example of the recombinant vector of the present disclosure is a Ti-plasmid vector capable of transferring part of itself, the so-called T-region to a plant cell when present in a suitable host. Other types of Ti-plasmid vectors are currently used to transfer hybrid DNA sequences to plant cells or protoplasts which may produce new plants capable of properly inserting hybrid DNA into the genome of a plant. A particularly suitable form of the Ti-plasmid vector is a so-called binary vector as claimed in EP 0120 516 B1 and US 4,940,838. Other vectors suitable for use in introducing DNA according to the present disclosure into a plant host may be selected from viral vectors which may be derived from a double-stranded plant virus (e.g., CaMV), a single-stranded virus, a geminivirus, or the like, for example, incomplete plant viral vectors. Such vectors may be used especially when it is difficult to suitably transform a plant host.

Examples of the suitable vector may include, but are not limited to, 326 GFP- or pCAMBIA-based binary vectors, and any vector capable of expressing genes encoding the DNA fragment and carbonic anhydrase according to the present disclosure in a plant cell may be used by those of ordinary skill in the art.

More specifically, the recombinant vector is a recombinant expression vector based on an existing vector used for protein expression as a main skeleton to enhance the expression of a promoter and a target protein, in which the DNA fragment according to the present disclosure and a gene encoding the target protein are sequentially operably linked. In addition, the expression vector may include a ribosomal binding site as a translation initiation site and a transcription terminator. The expression vector preferably includes one or more selectable markers. The marker is typically a nucleic acid sequence having properties that can be selected by a chemical method, which corresponds to all genes that can distinguish transformed cells from non-transformed cells. Examples thereof include, but are not limited to, herbicide resistance genes such as glyphosate or phosphinothricin, antibiotic resistance genes such as kanamycin, G418, bleomycin, hygromycin, and chloramphenicol, and aadA genes.

The term "operably linked" may be intended to mean a gene and an expression regulator that are linked in such a way to enable gene expression when an appropriate molecule binds to the expression regulator.

In the recombinant vector of present disclosure, the promoter may be any one promoter selected from the group consisting of a 35S promoter derived from a cauliflower mosaic virus, a 19S RNA promoter derived from a cauliflower mosaic virus, an actin protein promoter of a plant, and an ubiquitin protein promoter, but the present disclosure is not limited thereto. The term "promoter" means a DNA upstream region from a structural gene, and refers to a DNA molecule to which an RNA polymerase binds to initiate transcription. A "plant promoter" is a promoter capable of initiating transcription in plant cells. A "constitutive promoter" is a promoter that is active under most environmental conditions and developmental conditions or cell differentiation. In the present disclosure, a constitutive promoter is preferable since the selection of a transformant may be made by various tissues in various processes. Thus, constitutive promoters may be selected without limitation.

In the recombinant vector of the present disclosure, a conventional terminator may be used, and examples thereof include, but are not limited to, a nopaline synthase (NOS) terminator, a rice amylase RAmy1 A terminator, a phaseolin terminator, a terminator of the octopine gene of Agrobacterium tumafaciens, and an rrnB 1B2 terminator of Escherichia coli.

The above-described recombinant vector of the present disclosure is illustrated with a cleavage map in FIG. 1A, 1D, 2A, or 2D.

The present disclosure may also provide a transformant transformed with the recombinant vector.

In addition, when transforming the vector of the present disclosure into an eukaryotic cell, yeast (Saccharomyce cerevisiae), an insect cell, a human cell (e.g., a CHO cell line (Chinese hamster ovary), W138, BHK, COS-7, 293, HepG2, 3T3, RIN, and an MDCK cell line), a plant cell, and the like may be used as a host cell, and the host cell may be preferably Agrobacterium, more preferably *Agrobacterium tumefaciens,* and more particularly, may be Agrobacterium LBA4404, GV3101, AGL1, EHA101 or EHA105.

When the host cell is a prokaryotic cell, a method of delivering the vector of the present disclosure into the host cell may be performed using a $CaCl_2$ method, a method by Hanahan (Hanahan, D., J. Mol. Biol., 166:557-580(1983)), an electroporation method, and the like. In addition, when the host cell is a eukaryotic cell, the vector may be injected into the host cell by microinjection, calcium phosphate precipitation, electroporation, liposome-mediated transfection, DEAE-dextran treatment, gene bombardment, or the like.

The recombinant vector may be used in a method of producing a carbonic anhydrase in a plant, including: (a) constructing the above-described recombinant vector; (b) introducing the recombinant vector into a cell to prepare a transformant; (c) culturing the transformant; (d) infiltrating the culture solution into a plant; and (e) pulverizing the plant and causing the pulverized plant to bind to cellulose beads.

The method of producing a carbonic anhydrase from a transformed plant cell may be performed by transforming a plant cell with the recombinant vector according to the present disclosure, and then culturing the plant cell for an appropriate period of time to cause the carbonic anhydrase to be expressed and obtaining the carbonic anhydrase from the transformed cell. In this case, the carbonic anhydrase may be expressed using any method known in the art.

The cellulose bead may be any one cellulose bead selected from the group consisting of a carboxyl methyl cellulose (CMC) bead, a methyl cellulose (MC) bead, an amorphous cellulose (AMC) bead, a hydroxyl ethyl cellulose (HEC) bead, and a hydroxy propyl methyl cellulose (HPMC) bead, and may be, most preferably, an AMC bead.

In (d) infiltrating the culture solution into a plant, a culture of a recombinant vector including P38 in addition to the culture of the recombinant vector for producing a carbonic anhydrase of the present disclosure may be simultaneously infiltrated. p38 is a gene silencing suppressor. In addition, gene silencing suppressor P19, HC-Pro, TVA 2b, or the like may be used.

The infiltration into a plant may be performed by a chemical cell method or a vacuum or syringe infiltration method, most preferably a syringe infiltration method.

The plant may be selected from: food crops including rice, wheat, barley, corn, soybeans, potatoes, red beans, oats, and sorghum; vegetable crops including Arabidopsis, Chinese cabbage, radish, peppers, strawberries, tomatoes, watermelons, cucumbers, cabbages, melons, pumpkins, green onions, onions, and carrots; special crops including ginseng, tobacco, cotton, sesame, sugar cane, sugar beet, perilla, peanuts, and rapeseed; fruit trees including apple trees, pear trees, jujube trees, peaches, grapes, citrus fruits, persimmons, plums, apricots, and bananas; flowers including roses, carnations, chrysanthemums, lilies, and tulips.

In (e) pulverizing the plant and causing the pulverized plant to cellulose beads, a ratio of an extract obtained by pulverizing the plant and the cellulose beads may range from about 1:10 to about 2:1, preferably about 1:10 to about 1:1, and most preferably 4:10.

The inventors of the present disclosure determined the binding capacity of CBM3 to 10 mg of cellulose beads using various amounts of (1 $\mu$l to 20 $\mu$l) of a total *N. benthamiana* leaf extract. As a result, when 4 $\mu$l of the total extract was mixed with cellulose beads, a trace amount of a CBM3 band began to be shown in an unbound region in the case of MSC-GcCAH$\alpha$3, and at 10 $\mu$l and 20 $\mu$l, about a half amount of the CBM3 band was shown in the unbound region. From these results, the amount of CBM3 in 4 $\mu$l of the total protein extract was determined as an amount that slightly exceeded saturation of the cellulose beads. When up to 2 $\mu$l of the total extract was mixed with cellulose beads, the CBM3 band was not shown in an unbound region in the case of MSC-GcCAH$\alpha$3-M, and at 4 $\mu$l and 10 $\mu$l, about a half amount of CBM3 was shown in the unbound region (see FIG. 3). When up to 20 $\mu$l of the total protein extract was mixed with cellulose beads, the CMB3 band was not shown in an unbound region in the case of MSC-Saz and MSC-Saz-M.

The carbonic anhydrase includes CBM3, the CBM3 can induce a firm bond with the surfaces of the cellulose beads, which induces the hydration reaction of carbon dioxide, thereby collecting carbon dioxide (see FIGS. 9 and 10).

The recombinant vector may be used in a method of collecting carbon dioxide, including: preparing a composition for collecting carbon dioxide; and supplying carbon dioxide to the composition for collecting carbon dioxide.

The preparation of the composition for collecting carbon dioxide may include: (a) constructing the above-described recombinant vector; (b) introducing the recombinant vector into a cell to prepare a transformant; (c) culturing the transformant; (d) infiltrating the culture solution into a plant; and (e) pulverizing the plant and causing the pulverized plant to bind to cellulose beads.

The method for supplying carbon dioxide to the composition for collecting carbon dioxide is not particularly limited, and carbon dioxide may be supplied in the form of a source containing a large amount of carbon dioxide and in need of the removal of carbon dioxide, for example, wastewater or flue gas.

In addition, as described above, after collecting carbon dioxide using the composition for collecting carbon dioxide, a metal cation source may be added to finally convert the collected carbon dioxide into a carbonate or bicarbonate precipitate to be variously used industrially. In addition, through the dehydration of a bicarbonate solution, $CO_2$ may be purely produced again.

As used herein, the term "carbonate" or "carbonate precipitate" means an inorganic component generally containing the carbonate group $CO_3$. The term may include both mixtures of carbonates and bicarbonates and species containing only carbonate ions. In addition, the term "bicarbonate" or "bicarbonate precipitate" generally means an inorganic component containing the bicarbonate group $HCO_3$. The term may include both mixtures of carbonates and bicarbonates and species containing only bicarbonate ions.

The metal ion source supplied to react with carbon dioxide to produce a carbonate or a bicarbonate is not particularly limited as long as it contains metal ions, and may be appropriately selected depending on the intended use, but may preferably react with a carbonate source and those capable of forming carbonates having a calcite crystalline form, an aragonite crystalline form, a vaterite crystalline form, or an amorphous crystalline form may be used.

For example, $Na^+$ ions, $Ca^{2+}$ ions, $Fe^{2+}$ ions, $Mn^{2+}$ ions, $Sr^{2+}$ ions, $Ba^{2+}$ ions, $Zn^{2+}$ ions, or $Pb^{2+}$ ions may be used as the metal ion source, and nitrates, hydrochlorides, hydroxides, or basic solutions including the same may be used.

The recombinant vector may be used in the preparation of a composition for liberating carbon dioxide, which preparation may include: (a) constructing the above-described recombinant vector; (b) introducing the recombinant vector into a cell to prepare a transformant; (c) culturing the transformant; (d) infiltrating the culture solution into a plant; and (e) pulverizing the plant and causing the pulverized plant to bind to cellulose beads.

**[Example 1]**

**Construction of MSC-GcCAH$\alpha$3 (Recombinant Fusion Construct of BiP:M:SUMO:GcCAH$\alpha$3:CBM3:HDEH) and MSC-SazCA ((BiP:M:SUMO:SazCA:CBM3:HDEH)**

MSC-GcCAH$\alpha$3 was designed as follows. BiP, an ER targeting signal, was used to induce GcCAH$\alpha$3 expression

in the ER to be accumulated at a high level. An M domain that enhances protein expression was linked to BiP. Fusion was performed to have bdSUMO which enhances the solubility of a protein. GcCAHα3 was fused. Finally, fused CBM3-HDEL was obtained. CBM3 is a cellulose-binding domain used for protein separation, and HDEL at the C-terminal induces high accumulation in the ER. The translation amplification sequence 5'-UTR was included in the recombinant fusion gene (BiP:M:SUMO:GcCAHα3:CBM3:HDEH, MSC-GcCAHα3) to increase a translation level. As a promoter and terminator of the recombinant fusion gene, a CaMV 35S promoter and an HSP terminator were used to thereby complete a binary vector for expression. As a backbone of the binary vector, pCAMBIA1300 was used (see FIG. 1A). The SEQ ID NO of the gene is shown in Table 1 below. The binary vector designed as described above was constructed as follows. CBM3 (AEI55081) and bdSUMO (amino acids 21-97) DNA fragments were chemically synthesized (Bioneer Corp., Daejeon, Korea). All synthesized DNA was optimized for codons to fit *Nicotiana benthamiana*. Recombinant gene MSC-GcCAHα3 was constructed by overlapping PCR. His-Asp-Glu-Leu (HDEL), which is an ER retention signal, was added to the C-terminal of a reverse primer used during the overlapping PCR. The final PCR product was cleaved with BamHI and XhoI restriction enzymes, and the plant expression vector 326-GFP was cleaved with BamHI and XhoI and then ligated. The M domain of MSC-GcCAHα3 (N-terminal fragment of human protein tyrosine phosphatase receptor type C (CD45), amino acid positions 231 to 290) was introduced through overlapping PCR using plasmid EelepfM as a template. The resulting plasmid was named 326-M-bdSUMO-GcCAHα3-CBM3-HDEL. The plasmid was cleaved with XbaI and EcoRI to obtain fragments, and the binary vector pCAMBIA1300 was cleaved with XbaI and EcoRI, followed by ligation, thereby completing the construction of MSC-GcCAHα3 (see FIG. 1A). A double enhancer version (d35S) of a cauliflower mosaic virus (CaMV) 35S promoter was used as a promoter in MSC-GcCAHα3. In addition, the 5' untranslated enhancer region (5'UTR) and the leader sequence (amino acids 1 to 34) of *Arabidopsis thaliana* BiP1 (BAA13947) was added to the 5' end, and HSP18 of *Arabidopsis thaliana* was used as a terminator. The plasmid DNA was identified by sequencing using a 35s promoter (5'-GAAGACGTTCCAACCACGTC-3') and an HSP terminator primer (5'-CGGAATTCCTTATCTTTAATCATATTCC-3'). A SazCA binary expression vector was constructed in the same manner as for MSC-GcCAHα3 (see FIG. 1D). SazCA optimized for codons was synthesized by a chemical method (Bioneer Corp. Daejeon, South Korea). The amino acid sequence and nucleotide sequence of SazCA are shown in FIG. 1. SazCA was introduced into *E. coli* and amplified for use. To construct MC-SazCA, BiP and an M domain were first obtained by PCR. The 5' end of the PCR product was allowed to have an XbaI restriction enzyme site and have a SpeI restriction enzyme site at the 3' end of the M-domain. The PCR product was introduced on the 5' side of CBM of CBM3-HDEL in a 326-sGFP vector. SazCA was then introduced using the SpeI and KpnI restriction sites between the M-domain and CBM3. Finally, BiP:M:SazCA:CBM3:HDEL was obtained using restriction enzymes XbaI and EcoRI and ligated to pCAMBIA 1300 treated with the same type of restriction enzymes to construct a binary vector. The obtained clones were identified by sequencing using a 35s promoter primer (5'-GAAGACGTTCCAACCACGTC-3') and a HSP terminator primer (5'-CGGAATTCCTTATCTTTAATCATATTCC-3').

[Table 1]

| Gene | Sequence | SEQ ID NO: |
|---|---|---|
| GcCAHα3 (Amino acid) | MNFFATASVFLLFLSVALPVRANEEIEVDTAGCPVKSA QGDFSYDMTLPNNPTRWGDIKEDFATCKEGEKQSPINF PANVQYAPKSAGPKSQMSLANMTFGASSYNWAMSCS DESGSCGKTSFAGKTYELINVHFHSPSEHKLFGKEYPL ECHMVHAAEDGSLAVVGMMFEYAEQTSYPARIYQNV VEEYGDNVVFSTILGGVKSDRGEWAVPVGQLVNHNK GYCSYSGSLTTPPCTESVTWFMSMNIWTVSERQVHDY VRTVGTSIEGNHRPVQPLNERPVTCYVS | SEQ ID NO: 1 |

(continued)

| Gene | Sequence | SEQ ID NO: |
|------|----------|------------|
| GcCAHα3 (CDS) | ATGAACTTCTTCGCTACCGCCTCCGTCTTTCTACTCT TCTTGTCCGTCGCCCTCCCCGTGCGGGCGAACGAGG AAATCGAGGTCGACACCGCCGGCTGCCCCGTCAAGA GCGCCCAAGGAGACTTCAGTTATGACATGACTCTTC CAAACAACCCCACCCGCTGGGGCGACATCAAGGAG GACTTCGCCACATGCAAGGAAGGAGAAAAGCAATC GCCCATCAACTTCCCCGCCAACGTTCAATACGCCCC GAAATCGGCCGGACCCAAGTCTCAAATGTCGCTCGC AAACATGACCTTTGGCGCCTCCTCCTACAACTGGGC CATGTCCTGCAGCGACGAATCCGGATCGTGTGGAAA GACCTCCTTCGCTGGAAAGACCTATGAACTTATCAA TGTTCACTTCCACTCCCCGAGTGAGCACAAGTTGTTC GGCAAAGAATATCCGCTTGAATGTCACATGGTGCAT GCTGCCGAGGATGGATCGCTTGCTGTGGTCGGAATG ATGTTTGAGTACGCCGAACAAACCTCCTATCCCGCC AGAATTTACCAGAACGTCGTTGAGGAATATGGCGAC AACGTCGTCTTTTCCACTATTTTGGGAGGCGTCAAG AGCGATCGTGGAGAATGGGCTGTGCCGGTGGGTCA ACTGGTCAACCATAACAAGGGATATTGCTCCTACTC | SEQ ID NO: 2 |
| | CGGGTCGCTCACCACACCGCCTTGCACTGAAAGTGT CACCTGGTTCATGTCTATGAACATCTGGACCGTGTCT GAACGACAGGTGCATGACTACGTCCGAACGGTTGGC ACCAGCATTGAGGGAAATCATCGACCGGTTCAACCA CTCAACGAACGTCCGGTCACTTGCTACGTGTCCTAA | |
| 5'UTR | TCTAGAATTATTACATCAAAACAAAAA | SEQ ID NO: 3 |
| BIP | ATGGCTCGCTCGTTTGGAGCTAACAGTACCGTTGTG TTGGCGATCATCTTCTTCGGTGAGTGATTTTCCGATC TTCTTCTCCGATTTAGATCTCCTCTACATTGTTGCTT AATCTCAGAACCTTTTTTCGTTGTTCCTGGATCTGAA TGTGTTTGTTTGCAATTTCACGATCTTAAAAGGTTAG ATCTCGATTGGTATTGACGATTGGAATCTTTACGATT TCAGGATGTTTATTTGCGTTGTCCTCTGCAATAGAAG AGGCTACGAAGTTAA | SEQ ID NO: 4 |
| M domain (Amino acid) | ANITVDYLYNKETKLFTAKLNVNENVECGNNTCTNNE VHNLTECKNASVSISHNSCTAPD | SEQ ID NO: 5 |
| M domain | GGATCCCGATGGCAAACATCACTGTGGATTACTTAT ATAACAAGGAAACTAAATTATTTACAGCAAAGCTAA ATGTTAATGAGAATGTGGAATGTGGAAACAATACTT GCACAAACAATGAGGTGCATAACCTTACAGAATGTA AAAATGCGTCTGTTTCCATATCTCATAATTCATGTAC TGCTCCTGATGGTGGAGGAGGTTCTGGTGGTGGATC AACTAGTCATATT | SEQ ID NO: 6 |

(continued)

| Gene | Sequence | SEQ ID NO: |
|------|----------|------------|
| SUMO | AATTTGAAAGTGAAGGGACAGGACGGAAACGAGGT TTTCTTCCGTATCAAAAGATCAACACAACTCAAGAA ACTCATGAATGCTTACTGCGATAGACAAAGCGTGGA CATGACAGCTATAGCTTTCCTATTTGATGGGAGAAG GTTGAGAGCGGAACAGACTCCGGATGAGCTTGAAA TGGAAGATGGAGATGAGATTGACGCAATGTTACATC AGACTGGTGCCGGCGGTCCCGGGGGT | SEQ ID NO: 7 |
| CBM 3 | GGAGGAGGTTCAGGTGGTACCGTATCAGGTAACCTT AAGGTGGAGTTTTACAACTCGAACCCTTCTGATACA ACTAACTCAATAAACCCACAGTTCAAAGTTACAAAC ACAGGCAGCTCTGCGATCGATTTGTCTAAATTAACC CTCAGATACTATTATACGGTTGATGGACAGAAGGAC CAGACTTTCTGGTGTGATCATGCAGCTATCATTGGTT CTAACGGTAGCTACAACGGAATTACATCAAACGTGA AGGGCACTTTCGTTAAGATGTCCTCTAGCACTAACA ACGCAGACACATATTTGGAGATCAGTTTTACGGGGG GAACCCTTGAACCAGGTGCTCACGTCCAGATTCAAG | SEQ ID NO: 8 |
| | GAAGATTCGCTAAAAACGACTGGTCGAACTATACCC AAAGTAATGATTACAGTTTTAAATCCGCCTCACAAT TTGTTGAGTGGGATCAGGTCACTGCTTACCTGAACG GGGTTCTAGTGTGGGGAAAGGAACCTGGT | |
| HDEL | CATGACGAGCTT | SEQ ID NO: 9 |

(continued)

| Gene | Sequence | SEQ ID NO: |
|------|----------|-----------|
| FULL EXPRESSI ON CASSETT E (FULL SEQUENC E): BIP_M_GC CAHa3 _CBM3_H DEL | tctagaattattacatcaaaacaaaaaatggctcgctcgtttggagctaacagtaccgtt gtgttggcgatcatcttcttcggtgagtgattttccgatcttcttctccgatttagatctcctc tacattgttgcttaatctcagaaccttttttcgttgttcctggatctgaatgtgtttgtttgcaat ttcacgatcttaaaaggttagatctcgattggtattgacgattggaatctttacgatttcag gatgtttatttgcgttgtcctctgcaatagaagaggctacgaagttaaggatcccgatgg caaacatcactgtggattacttatataacaaggaaactaaattatttacagcaaagctaaa tgttaatgagaatgtggaatgtggaaacaatacttgcacaaacaatgaggtgcataacc ttacagaatgtaaaaatgcgtctgtttccatatctcataattcatgtactgctcctgatggtg gaggaggttctggtggtggatcaactagtcatattaatttgaaagtgaagggacaggac ggaaacgaggttttcttccgtatcaaaagatcaacacaactcaagaaactcatgaatgc ttactgcgatagacaaagcgtggacatgacagctatagctttcctatttgatgggagaa ggttgagagcggaacagactccggatgagcttgaaatggaagatggagatgagattg acgcaatgttacatcagactggtgccggcggtcccggggggtatgaacttcttcgctacc gcctccgtctttctactcttcttgtccgtcgccctccccgtgcgggcgaacgaggaaatc gaggtcgacaccgccggctgccccgtcaagagcgcccaaggagacttcagttatga catgactcttccaaacaaccccacccgctggggcgacatcaaggaggacttcgccac atgcaaggaaggagaaaagcaatcgcccatcaacttccccgccaacgttcaatacgc cccgaaatcggccggaacccaagtctcaaatgtcgctcgcaaacatgacctttggcgcc tcctcctacaactgggccatgtcctgcagcgacgaatccggatcgtgtggaaagacct ccttcgctggaaagacctatgaacttatcaatgttcacttccactcccccgagtgagcaca agttgttcggcaaagaatatccgcttgaatgtcacatggtgcatgctgccgaggatgga tcgcttgctgtggtcggaatgatgtttgagtacgccgaacaaacctcctatcccgccag aatttaccagaacgtcgttgaggaatatggcgacaacgtcgtcttttccactattttggga ggcgtcaagagcgatcgtggagaatgggctgtgccggtggggtcaactggtcaaccat aacaagggatattgctcctactccgggtcgctcaccacaccgccttgcactgaaagtgt cacctggttcatgtctatgaacatctggaccgtgtctgaacgacaggtgcatgactacgt ccgaacggttggcaccagcattgagggaaatcatcgaccggttcaaccactcaacga acgtccggtcacttgctacgtgtcctaaggaggaggttcaggtggtaccgtatcaggta accttaaggtggagttttacaactcgaacccttctgatacaactaactcaataaacccac agttcaaagttacaaacacaggcagctctgcgatcgatttgtctaaattaaccctcagat actattatacggttgatggacagaaggaccagacttctggtgtgatcatgcagctatca ttggttctaacggtagctacaacggaattacatcaaacgtgaagggcactttcgttaaga tgtcctctagcactaacaacgcagacacatatttggagatcagttttacggggggggaacc cttgaaccaggtgctcacgtccagattcaaggaagattcgctaaaaacgactggtcga actatacccaaagtaatgattacagttttaaatccgcctcacaatttgttgagtgggatca ggtcactgcttacctgaacggggttctagtgtggggaaaggaacctggtcatgacgag ctttaactcgag | SEQ ID NO: 10 |
| SazCA (Amino acid sequence) | MKKFILSILSLSIVSIAGEHAILQKNAEVHHWSYEGENG PENWAKLNPEYFWCNLKNQSPVDISDNYKVHAKLEK LHINYNKAVNPEIVNNGHTIQVNVLEDFKLNIKGKEYH LKQFHFHAPSEHTVNGKYYPLEMHLVHKDKDGNIAVI GVFFKEGKANPELDKVFKNALKEEGSKVFDGSININAL | SEQ ID NO: 11 |
| | LPPVKNYYTYSGSLTTPPCTEGVLWIVLKQPITASKQQI ELFKSIMKHNNNRPTQPINSRYILESN | |

(continued)

| Gene | Sequence | SEQ ID NO: |
|------|----------|------------|
| SazCA | ATGAAAAAATTCATACTGAGCATACTCAGTCTATCC ATCGTCTCAATAGCGGGAGAGCATGCCATTCTGCAA AAAAATGCAGAGGTACATCACTGGTCATATGAAGGT GAAAACGGTCCAGAGAATTGGGCAAAATTGAACCC GGAATATTTTGGTGCAACCTGAAGAATCAGTCTCC CGTGGATATATCTGACAATTATAAGGTGCACGCAAA GCTGGAAAAATTGCATATCAACTACAATAAAGCAGT CAATCCTGAGATAGTGAATAATGGGCACACAATTCA GGTGAATGTCCTTGAGGACTTTAAGCTAAATATAAA AGGAAAGAGTATCATCTCAAACAGTTCCATTTCCA TGCACCGAGTGAACACACCGTCAACGGAAAATACT ATCCCTTGGAGATGCACCTGGTACATAAAGATAAAG ACGGTAACATCGCTGTCATCGGGGTTTTTTTAAGG AAGGTAAGGCGAACCCAGAACTCGATAAAGTCTTC AAAAACGCCCTGAAGGAAGAGGGCTCTAAGGTCTT CGACGGAAGTATAAACATCAACGCTTTACTTCCGCC CGTGAAGAACTACTATCGTACAGCGGCTCACTGAC AACTCCGCCGTGCACTGAAGGAGTTTTATGGATTGT ACTGAAGCAGCCGATTACCGCTTCCAAGCAGCAAAT AGAACTCTTTAAGTCAATAATGAAGCATAATAACAA TCGACCCACGCAACCTATCAATAGCCGATACATCTT GGAATCTAA | SEQ ID NO: 12 |
| BIP_M_SA ZCA_CBM 3_HDEL | TCTAGAATTATTACATCAAAACAAAAAATGGCTCGC TCGTTTGGAGCTAACAGTACCGTTGTGTTGGCGATC ATCTTCTTCGGTGAGTGATTTTCCGATCTTCTTCTCC GATTTAGATCTCCTCTACATTGTTGCTTAATCTCAGA ACCTTTTTTCGTTGTTCCTGGATCTGAATGTGTTTGT TTGCAATTTCACGATCTTAAAAGGTTAGATCTCGATT GGTATTGACGATTGGAATCTTTACGATTTCAGGATG TTTATTTGCGTTGTCCTCTGCAATAGAAGAGGCTAC GAAGTTAA GGATCCCGATGGCAAACATCACTGTGGATTACTTAT ATAACAAGGAAACTAAATTATTTACAGCAAAGCTAA ATGTTAATGAGAATGTGGAATGTGGAAACAATACTT GCACAAACAATGAGGTGCATAACCTTACAGAATGTA AAAATGCGTCTGTTTCCATATCTCATAATTCATGTAC TGCTCCTGATGGTGGAGGAGGTTCTGGTGGTGGATC AACTAGT ATGAAAAAATTCATACTGAGCATACTCAGTCTATCC ATCGTCTCAATAGCGGGAGAGCATGCCATTCTGCAA AAAAATGCAGAGGTACATCACTGGTCATATGAAGGT GAAAACGGTCCAGAGAATTGGGCAAAATTGAACCC GGAATATTTTGGTGCAACCTGAAGAATCAGTCTCC CGTGGATATATCTGACAATTATAAGGTGCACGCAAA GCTGGAAAAATTGCATATCAACTACAATAAAGCAGT CAATCCTGAGATAGTGAATAATGGGCACACAATTCA GGTGAATGTCCTTGAGGACTTTAAGCTAAATATAAA | SEQ ID NO: 13 |

(continued)

| Gene | Sequence | SEQ ID NO: |
|---|---|---|
| | AGGAAAAGAGTATCATCTCAAACAGTTCCATTTCCA TGCACCGAGTGAACACACCGTCAACGGAAAATACT ATCCCTTGGAGATGCACCTGGTACATAAAGATAAAG ACGGTAACATCGCTGTCATCGGGGTTTTTTTTAAGG AAGGTAAGGCGAACCCAGAACTCGATAAAGTCTTC AAAAACGCCCTGAAGGAAGAGGGCTCTAAGGTCTT CGACGGAAGTATAAACATCAACGCTTTACTTCCGCC CGTGAAGAACTACTATCGTACAGCGGCTCACTGAC AACTCCGCCGTGCACTGAAGGAGTTTTATGGATTGT ACTGAAGCAGCCGATTACCGCTTCCAAGCAGCAAAT AGAACTCTTTAAGTCAATAATGAAGCATAATAACAA TCGACCCACGCAACCTATCAATAGCCGATACATCTT GGAATCTAATGGAGGAGGTTCAGGTGGTACCGTATC AGGTAACCTTAAGGTGGAGTTTTACAACTCGAACCC TTCTGATACAACTAACTCAATAAACCCACAGTTCAA AGTTACAAACACAGGCAGCTCTGCGATCGATTTGTC TAAATTAACCCTCAGATACTATTATCGGTTGATGG ACAGAAGGACCAGACTTTCTGGTGTGATCATGCAGC TATCATTGGTTCTAACGGTAGCTACAACGGAATTAC ATCAAACGTGAAGGGCACTTTCGTTAAGATGTCCTC TAGCACTAACAACGCAGACACATATTTGGAGATCAG TTTTACGGGGGGAACCCTTGAACCAGGTGCTCACGT CCAGATTCAAGGAAGATTCGCTAAAAACGACTGGTC GAACTATACCCAAAGTAATGATTACAGTTTTAAATC CGCCTCACAATTTGTTGAGTGGGATCAGGTCACTGC TTACCTGAACGGGGTTCTAGTGTGGGGAAAGGAACC TGGTCATGACGAGCTTTAACTCGAG | |

**[Example 2]**

**Construction of MSC-GcCAHα3-M (Recombinant Fusion Construct of BiP:M:SUMO:GcCAHα3:M:CBM3:HDEL) and MSC-SazCA-M ((BiP:M:SUMO:SazCA:M:CBM3: HDEL)**

An M domain was amplified along with CBM3 by two primers to produce MSC-GcCAHα3-M (recombinant fusion compound of BiP:M:SUMO:GcCAHα3:M:CBM3:HDEL) (at Kpn1 restriction enzyme site for forward primer and at Xho1 restriction enzyme site for reverse primer), and PCR was performed using p1300-C3bdSU-hIL6 DNA (Islam et al., 2018) as a template. The amplified fragment was subcloned into a 326-M-bdSUMO-GcCAHα3-CBM3-HDEL-HSPt vector. The constructed plasmid was named 326-M-bdSUMO-ML-CBM3-HDEL-HSPt, digested with XbaI and EcoRI, inserted into the plant expression binary vector pCAMBIA1300 digested with the same restriction enzymes Xba I and EcoRI, and the resulting construct was named pCAMBIA1300-M-bdSUMO-ML-CBM3-HDEL-HSPt. Finally, MSC-GcCAHα3-M was constructed by cleaving pCAMBIA1300-M-bdSUMO-ML-CBM3-HDEL-HSPt with Xma1 and Kpn1 restriction enzymes and inserting GcCAHα3 between bdSUMO and the M domain. To construct a MSC-SazCA-M construct, BiP:M:SUMO:SazCA:CBM3:HDEH was cleaved with XmaI and KpnI restriction enzymes to obtain a SazCA segment. The SazCA segment was inserted into MSC-GcCAHα3-M cleaved with the same restriction enzymes so that SazCA was located between bdSUMO and the M domain, thereby completing the construction of MSC-SazCA-M.

**[Example 3]**

**Introduction of Plasmid into Agrobacterium strains GV3101 and EHA105**

Each of the MSC-GcCAHα3, MSC-Saz, MSC-GcCAHα3-M, and MSC-Saz-M prepared according to Examples 1 and 2 was transfected into Agrobacterium strains GV3101 and EHA105. Plasmid DNA was transfected into Agrobacterium by electroporation and cultured on LB plates containing kanamycin and rifampicin (50 mg/L and 100 mg/L, respectively) at 28 °C for 2 days. A colony was selected and incubated overnight in LB media containing 5 mL of kanamycin and rifampicin. The prepared culture media were used to prepare a 50 mL LB culture solution for syringe infiltration or 400 mL LB culture solution for vacuum infiltration.

**[Example 4]**

**Expression of MSC-GcCAHα3, MSC-Saz, MSC-GcCAHα3-M, and MSC-Saz-M in *Nicotiana benthamiana***

Wild-type *N. benthamiana* was cultivated for Agrobacterium-mediated infiltration. Seeds of a plant were grown at 24 ° C and a relative humidity of 40-65% under long day conditions (14 h light/10 h dark) with a light intensity of 130-150 μE m-2 sec-1. A plant grown for 6 weeks was used for Agrobacterium-mediated infiltration. 1 mL of the Agrobacterium culture solution of Example 3 was added to a 50 ml LB culture solution comprising kanamycin and rifampicin (50 mg/L and 100 mg/L, respectively). After incubation for 16 hours, the Acrobacterium was recovered by centrifugation at 4000 × g condition at 28 °C for 8 minutes. The supernatant was discarded and the Acrobacterium precipitate was dissolved in an infiltration buffer (10 mM MES, 10 mM $MgSO_4$. $7H_2O$; pH 5.7). The concentration of cells was adjusted to 0.8 at OD600 using an infiltration buffer. Then, 400 μM acetosyringone was added to the Agrobacterium solution and left at room temperature for 2 hours. Infiltration with a syringe was performed using a 1 mL syringe without a needle. At the same time, a binary vector containing P38, a gene of the coat protein of a turnip crinkle virus, which is a gene silencing suppression gene, was co-infiltrated. To this end, the MSC-GcCAHα3, MSC-Saz, MSC-GcCAHα3-M, or MSC-Saz-M recombinant gene and P38 were introduced into each Agrobacterium, and then mixed with an appropriate concentration of Agrobacterium culture solution at a ratio of 1: 1, followed by infiltration into *N. benthamiana* leaves. On days 3, 5, and 7 after infiltration, a total protein extract was obtained from *N. benthamiana* leaf tissue, followed by western blot analysis, to identify expression levels. The total protein extract was prepared using an extraction buffer (50 mM Tris pH 7.5, 150 mM NaCl, 0.1% Triton X100, 2 mM DTT and 1% protease inhibitor cocktail), and 5 × sample buffer (250 mM Tris-HCl [pH 6.8], 10% SDS, 0.5% bromophenol blue, 50% glycerol v/v, and 500 mM DTT) was added thereto at a final concentration of 1 ×, thereby producing an electrophoretic protein sample. The protein extract sample was boiled for 5 minutes, then developed through 10% SDS-PAGE, and then subjected to western blot analysis using an anti-CBM3 antibody. As a result, MSC-GcCAHα3 showed no significant difference in expression between the protein extracts obtained on days 3, 5 and 7. However, there was a difference according to the Agrobacterium strain, and EHA105 showed a higher expression level (see FIG. 1C). MSC-Saz also showed no significant difference between the protein extracts obtained on days 3, 5 and 7, and there was no difference according to the Agrobacterium strain. A strong band appeared at the position below the 66 kD band, and a weak band was identified in the samples at 5 and 7 days at the 116 kD position, which had an about two-fold size. This was assumed to be a protein corresponding to a dimer (see FIG. 1F). MSC-GcCAHα3-M showed higher expression of protein extracts obtained on day 5 than on days 3 and 7, according to the Agrobacterium strain, and EHA105 showed higher expression (see FIG. 2C). MSC-Saz-M also showed a similar trend with MSC-Saz (see FIG. 2F). From the above results, it was confirmed that, in fusion of the M domain to a vector, fusion by dividing into M1 and M2 further increased protein expression. It was confirmed that Agrobacterium EHA105 showed higher expression.

**[Example 5]**

**Pure Separation of Recombinant Proteins MSC-GcCAHα3, MSC-Saz, MSC-GcCAHα3-M, and MSC-Saz-M Using Cellulose Beads**

CBM3 has a high binding affinity for cellulose. Using this property, CBM3 was used as an affinity resin for protein separation. *N. benthamiana* leaf tissue was placed in liquid nitrogen and powdered using a mortar and pestle. 40 mL of extraction buffer (50 mM Tris pH 7.5, 150 mM NaCl, 0.1% Triton X100, 2 mM DTT and 1% protease inhibitor cocktail) was added to the leaf tissue powder (20 g). Samples were vortexed using a homogenate for 3-4 minutes to prepare extracts. Cell debris was removed by centrifugation at 14000 × g for 20 minutes. After centrifugation, 10 g of cellulose beads (500 μM average size) were added to the supernatant, followed by incubation at 4 °C for 1 hour. Proteins bound to cellulose beads were obtained by centrifugation at 1000 × g for 2 minutes. The cellulose beads were washed at least four times with a 40 mM Tris, pH 7.5, solution. The binding capacity of CBM3 to 10 mg of cellulose beads was determined using various amounts (1 $\mu$l to 20 $\mu$l) of a total *N. benthamiana* leaf extract including the recombinant proteins MSC-GcCAH$\alpha$3, MSC-Saz, MSC-GcCAH$\alpha$3-M, and MSC-Saz-M produced in a plant. A membrane was divided into a region bound to the cellulose beads and an unbound region and developed through SDS-PAGE, followed by western blot analysis using an anti-CBM3 antibody to determine the binding capacity of CBM3 to cellulose. As illustrated in FIG. 3, when 4 $\mu$l of the total extract was mixed with cellulose beads, a trace amount of a CBM3 band began to be shown in the unbound region in the case of MSC-GcCAH$\alpha$3, and at 10 $\mu$l and 20 $\mu$l, about a half amount of the CBM3 band was shown in the unbound region. From these results, the amount of CBM3 in 4 $\mu$l of the total protein extract was determined as an amount that slightly exceeded saturation of the cellulose beads. When up to 2 $\mu$l of the total extract was mixed with cellulose beads, the CBM3 band was not shown in an unbound region in the case of MSC-GcCAH$\alpha$3-M, and at 4 $\mu$l and 10 $\mu$l, about a half amount of CBM3 was shown in the unbound region (see FIG. 3). From these results, the amount of CBM3 in 2 $\mu$l of the total protein extract was determined as an amount that slightly exceeded saturation of the cellulose beads. When up to 20 $\mu$l of the total protein extract was mixed with cellulose beads, the CMB3 band was not shown in an unbound region in the case of MSC-Saz and MSC-Saz-M. From these results, the amount of CBM3 in 20 $\mu$l or more of the total protein extract was determined as an amount that saturates the cellulose beads.

This binding force was used to purely separate recombinant proteins including CBM3. Subsequently, pure separation was performed using CBM3 of MSC-GcCAH$\alpha$3, MSC-Saz, MSC-GcCAH$\alpha$3-M, and MSC-Saz-M and cellulose beads. The total leaf extract was mixed with cellulose beads and the unbound protein was removed. This was collected in a flow through fraction, and the cellulose beads were then washed four times using a washing solution. One to four washes were selected. In addition, MSC-GcCAH$\alpha$3, MSC-Saz, MSC-GcCAH$\alpha$3-M, and MSC-Saz-M tightly bound to cellulose beads were boiled and eluted to recruit eluted portions. These portions were separated via SDS-PAGE and western bloting analysis was performed using an anti-CBM3 antibody (see FIGS. 4 and 5). This confirmed that MSC-GcCAH$\alpha$3, MSC-Saz, MSC-GcCAH$\alpha$3-M, and MSC-Saz-M are tightly bound to cellulose beads and are almost purely separated and purified. According to the western blot results and Coomassie blue staining results, MSC-GcCAH$\alpha$3, MSC-Saz, MSC-GcCAH$\alpha$3-M, and MSC-Saz-M appeared at the 68 kD position, and also appeared as other bands with a larger size than that at the 116 kD position (see FIGS. 4 and 5). The protein at the 68 kD position is also present as a doublet, which is predicted by differential glycosylation of N-glycosylation sites present in an M domain and the like. In addition, bands above the 116 kD position were determined to be multimers. It is well known that carbonic anhydrases exist as multimers, such as tetramers.

**[Comparative Example 1]**

**Expression and Separation of Carbonic Anhydrase in *Escherichia Coli***

To prepare a control protein (GcCAH$\alpha$3 free form), a plasmid with the coding region of GcCAH$\alpha$3 was produced. The coding region of GcCAH$\alpha$3 without a signal sequence was amplified by PCR with designated primers (forward: 5'-cgggatccgaggaaatcgaggtcgac-3', reverse: 3'-ccgctcgagttaggacacgtagcaagt-5') using Gracilariopsis chorda genomic DNA as a template. The PCR product was inserted into the pRSET-A vector (Invitrogen) using BamHI and XhoI restriction enzymes. The pRSET-A vector contains a His6 tag at the N-terminal, and the resulting construct, His6: GcCAH$\alpha$3, was introduced into an *E. coli* BL21 (DE3) strain for protein expression. His6: GcCAH$\alpha$3-expressing plasmids were introduced into *E. coli* BL21 (DE3) via electroporation, and single colonies were incubated overnight in 5 mL LB liquid media. The next day, 5 mL of a culture solution was transferred to 200 mL LB liquid media containing ampicillin (100 $\mu$g/mL). When the culture solution reached an OD value of 0.6 at 600 nm (after 3-4 hours of incubation), the incubation temperature was lowered to 30 °C. *E. coli* was incubated overnight at 18 °C with IPTG at a final concentration of 1 mM with 0.1 mM ZnSO$_4$. Cells were then collected by centrifugation at 5,000 $\times$ g for 10 min at 4 °C and pellets were stored at -20 °C. The frozen pellet was thawed on ice for 15 minutes and dissolved in a solution including 5 ml of ice cold phosphate buffered saline (PBS; 140 mM NaCl, 2.7 mM KCl, 10 mM Na$_2$HPO$_4$, 1.8 mM KH$_2$PO$_4$) containing 1 mM phenylmethyl sulfonyl fluoride (PMSF), 1 mM dithiothreitol (DDT), and 1% protease inhibitors (1 $\mu$g/mL leupeptin and 1 $\mu$g/mL pepstatin). Bacterial cells were disrupted by sonication using an ultrasonic generator VC 505/VC 750 device (Sonics, Newtown, CT, USA) using a 3-5 sec pulse at 40% amplitude on ice for 5-10 minutes. A cell extract was separated by centrifugation at 14,000 $\times$ g for 20 minutes and the supernatant was collected. Ni$^{2+}$-NTA slurry (1 mL, 0.5 mL bed volume) was added to a 15 mL tube and centrifuged briefly. The supernatant was removed and 2 mL of lysis buffer was added to the tube. After mixing slowly, the Ni$^{2+}$-NTA resin was centrifuged again and the supernatant was discarded. Clear bacterial lysates were added to the Ni$^{2+}$-NTA resin and mixed gently by shaking at 200 $\times$ g for 60 minutes at 4 °C using a rotary shaker. The lysate-Ni$^{2+}$-NTA mixture was placed in a column covered with a bottom outlet. After removing the lower cap, the eluate was collected. The column was washed twice with wash buffer equal to 5 times the bed volume (2.5 mL) and then washed at least 4 times with wash buffer containing 30 mM imidazole. The protein was eluted with an elution buffer containing 250 mM imidazole. The eluted protein was divided into 5 tubes and used as a control protein for activity analysis.

To produce a free-form control protein for SazCA, SazCA fragments were amplified by PCR with primers designated (forward: 5'-gctggatccatgaaaaaattcatactgagc-3', reverse: 3'-gggccgaattcttaattagattccaagatgtatcggct-5') from the synthesized SazCA gene and the PCR product was inserted into a pRSET-A vector after cleavage with BamHI and EcoRI restriction enzymes (Invitrogen). The produced plasmid will contain the His6 tag at the N-terminal of SazCA. For the expression of His6: SazCA protein from the construct, the construct was introduced into an E. coli BL21 (DE3) strain using an electric shock method. Single colonies were obtained therefrom and incubated overnight in 5 mL IB liquid media in a shaker. The next day, 5 mL of the culture solution was transferred to 200 mL LB liquid media containing ampicillin (100 $\mu$g/mL). Once the culture reached an optical density of 0.6-0.8 at 600 nm (OD600) (after 3-4 hours of culture), the incubation temperature was lowered to 30 °C. IPTG was added with 0.1 mM $ZnSO_4$ to a final concentration of 1 mM and E. coli was incubated overnight at 18 °C. E. coli was then collected by centrifugation at 5,000 × g for 10 min at 4 °C and the pellet was stored at -20 °C. His6: SazCA from the frozen pellet was separated and purified in the same manner as the method of obtaining His6: GcCAH$\alpha$3 from E. coli as above.

**[Example 6]**

**Quantification of MSC-GcCAH$\alpha$3, MSC-Saz, MSC-GcCAH$\alpha$3-M, and MSC-Saz-M Produced in Plant**

To quantify MSC-GcCAH$\alpha$3, MSC-Saz, MSC-GcCAH$\alpha$3-M, and MSC-Saz-M, the proteins bound to cellulose beads were boiled and eluted. The MSC-GcCAH$\alpha$3, MSC-Saz, MSC-GcCAH$\alpha$3-M, and MSC-Saz-M bound to cellulose beads were eluted using 100% ethylene glycol (EG). The amounts thereof eluted with EG were accurately quantified using a Bradford assay. Thereafter, known amounts of MSC-GcCAH$\alpha$3, MSC-Saz, MSC-GcCAH$\alpha$3-M, and MSC-Saz-M were loaded and boiled, the eluted proteins were loaded in an amount ranging from 1 $\mu$l to 8 $\mu$l, followed by Western blot analysis to compare signal intensities, thereby quantifying the amount of each protein bounds to cellulose beads. Through this, the amount thereof expressed in a plant was estimated. From these results, it was confirmed that 100 $\mu$g of carbonic anhydrase was expressed at a protein level from 1 g of fresh weight of leaves (see FIG. 6).

**[Example 7]**

**Use of Recombinant MSC-GcCAH$\alpha$3, MSC-Saz, MSC-GcCAH$\alpha$3-M, and MSC-Saz-M Produced in Plant after Immobilized onto Cellulose Beads in Hydration Reaction of Carbon Dioxide and Confirmation of Thermal Stability Enhancement**

The hydration reaction of carbon dioxide can be induced using carbonic anhydrase under various conditions. Attaching carbonic anhydrase to the surface of a solid provides advantageous conditions in recovery for recycling, separation from reactants, and the like. To determine a degree to which carbonic anhydrase attached to the surface of cellulose beads induces the hydration reaction of carbon dioxide, the activity thereof was compared with that of free-form carbonic anhydrase separated and purified from E. coli. The two types of carbonic anhydrase showed almost no difference in the efficiency of hydration reaction of carbon dioxide. In addition, the most important property in the hydration reaction of $CO_2$ using carbonic anhydrase is thermal stability. Thus, to confirm the thermal stability of GcCAH$\alpha$3 separated and purified from recombinant MSC-GcCAH$\alpha$3, MSC-Saz, MSC-GcCAH$\alpha$3-M, and MSC-Saz-M in a state of being immobilized onto cellulose beads and produced in plants, stability thereof at 70 °C was examined. As a control, free-form GcCAH$\alpha$3 separated and purified from E. coli was used. The recombinant protein GcCAH$\alpha$3 immobilized onto cellulose beads was aliquoted and subjected to water bathing at 70°C using a water bath (Finemould Precision Ind. Co, Korea). The protein precipitate was removed by centrifugation at 10,000 × g and 4 °C for 5 minutes. The supernatant was stored at 4 °C until protein activity was measured, and the activity of the protein was measured using a Wilbur-Anderson method. In the Wilbur and Anderson (1948) method, the activity of carbonic anhydrase was determined by adding an aqueous $CO_2$-saturated solution to 20 mM Tris-HCl buffer (pH 8.3) at 0 °C, and then measuring the time (sec) taken for pH to decrease from 8.3 to 6.3. The time measured without the enzyme was denoted as Tb, and the time measured with the enzyme was denoted as Tc. To determine a blank value, 6.0 ml of cooled 20 mM Tris-HCl buffer, pH 8.3 was added to a 20 ml beaker and maintained at 0-4 °C for 1 hour, followed by recording of pH. $CO_2$-saturated water cooled using 4 ml of ice was added to Tris buffer. Immediately, the time taken for pH to change from 8.3 to 6.3 was recorded and denoted as a Tb value. Similarly, to measure the activity of the enzyme, 6.0 ml of ice-chilled 20 mM M Tris-HCl buffer, pH 8.3 was added to a 20 ml beaker and maintained at 0-4 °C for 1 hour, followed by recording of pH. 0.1 ml of a diluted enzyme solution was added thereto. After rapidly adding 4 ml of an aqueous $CO_2$-saturated solution thereto, the time taken for pH to drop from 8.3 to 6.3 was measured and denoted as a Tc value.

## Calculation of Enzymatic Activity

$$U=10\ ((T\_(b\ )/T\_(c\ )\ )-1)/(mg\ protein)$$

The residual activity (%) of each protein after heat treatment was obtained using a ratio of the activity of each protein after heat treatment to the activity thereof before heat treatment. GcCAHα3 separated and purified from the recombinant MSC-GcCAHα3, MSC-Saz, MSC-GcCAHα3-M, and MSC-Saz-M; and GcCAHα3 separated and purified from *E. coli* was continuously cultured at 70 °C to confirm the activity thereof every week. The activity of GcCAHα3 was measured using the hydration reaction of $CO_2$. As a result, the activity of GcCAHα3 separated and purified from MSC-GcCAHα3 and MSC-GcCAHα3-M increased with heating, and showed little difference from 1 to 2 weeks. However, those immobilized onto cellulose beads exhibited slightly low activity at 3 and 4 weeks, the activity was actually decreased slowly at 5 and 6 weeks, and the activity was reduced to an extent of activity in the case of no heat treatment at an initial stage at 6 weeks as finally confirmed. In contrast, the free-form GcCAHα3 exhibited rapidly reduced activity at 5 and 6 weeks, the activity thereof was reduced, at 5 weeks, up to 50% of that before heat treatment, and the free-form GcCAHα3 exhibited little activity at 6 weeks (see FIG. 7). Through this, it was confirmed that GcCAHα3 immobilized onto cellulose beads maintained thermal stability for a longer period of time. In addition, since there is a 2-fold difference in molecular weight between GcCAHα3 produced in a plant and GcCAHα3 produced from *E. coli,* the activity of proteins immobilized onto cellulose beads was expected to actually be about two times higher. Both GcCAHα3 separated and purified from MSC-Saz and GcCAHα3 separated and purified from MSC-Saz-M exhibited increased activity when heated at 70 °C, compared to unheated samples. When heated for 2 weeks, the activity was approximately doubled. The activity was maintained for 3 weeks with increased activity, and then lowered at 4 weeks and lowered to the activity level before first heating, at 6 weeks. The free form also showed a similar activity curve and the activity thereof was lower than the activity level before first heating at 5 weeks. Through this, it was confirmed that SazCA immobilized onto cellulose beads exhibited higher resistance to high temperature than that of the free from expressed from *E. coli* (see FIG. 7).

**[Example 8]**

**Durability of Recombinant MSC-GcCAHα3, MSC-Saz, MSC-GcCAHα3-M, and MSC-Saz-M Immobilized onto Cellulose Beads**

Proteins immobilized onto cellulose beads can be reused. Therefore, the degree of reuse of the activity of GcCAHα3 separated and purified from MSC-GcCAHα3, MSC-Saz, MSC-GcCAHα3-M, and MSC-Saz-M, which are recombinant proteins produced in a plant, was examined. As a control, free-form GcCAHα3 separated and purified from *E. coli* was used. 10 μg of each protein was added to $CO_2$-saturated water (4 mL) and mixed with 6 mL of 20 mM Tris buffer (pH 8.3) cooled with ice to induce reaction, the time taken for the pH to change from 8.3 to 6.3 was measured to measure the activity of the protein. The measured reaction time was converted into specific activity using a Wilbur-Anderson formula. The specific activity of free-form GcCAHα3 was 5722 Wilber-Anderson units (WAU)/mg, and the specific activity of GcCAHα3 separated and purified from MSC-GcCAHα3 or MSC-GcCAHα3-M was 5200 WAU/mg (see FIG. 8). The specific activity of free-form GcCAHα3 was 8200 Wilber-Anderson units (WAU)/mg, and the specific activity of GcCAHα3 separated and purified from MSC-Saz or MSC-Saz-MD was 8200 WAU/mg (see FIG. 8). This is because MSC-GcCAHα3 separated and purified from a plant has a molecular weight that is twice the molecular weight of a free form produced from *E. coli.* Thus, MSC-GcCAHα3 produced in a plant was estimated to have almost two-fold actual activity. After the reaction, cellulose beads having the protein bound thereto were recovered and a new identical reaction was induced, and even after repeating the reaction 10 times, there was completely no change in activity. Through this, MSC-GcCAHα3 immobilized onto cellulose beads are expected to be used for a long period of time. Subsequently, the actual possibility of continuous use of proteins bound to cellulose beads was examined. To this end, a reaction system as illustrated in FIG. 9 was constructed. A column was constructed using cellulose onto which GcCAHα3 separated and purified from MSC-GcCAHα3, MSC-Saz, MSC-GcCAHα3-M, and MSC-Saz-MD was immobilized, and $CO_2$-saturated water was continuously suppled thereto using an infusion pump. Then, a bicarbonate ($HCO_3$-) solution was collected below the column (see FIG. 9). In addition, 0.2 M Tris buffer (pH 10.5) containing 5 ml of 0.1 M $CaC_{l2}$ was added thereto to induce the formation of $CaCO_3$. $CO_2$ is converted into bicarbonate ions through the hydration reaction of GcCAHα3, which then instantaneously binds to $Ca^{2+}$ at a high pH such as pH 10.5, thereby forming $CaCO_3$. The precipitated $CaCO_3$ was recovered and dried at 60 °C for 4 hours to measure the weight thereof. The specific activity of GcCAHα3 separated and purified from MSC-GcCAHα3, MSC-Saz, MSC-GcCAHα3-M, and MSC-Saz-M and immobilized onto cellulose beads was measured for 40 days. Although the activity of GcCAHα3:CBM3 separated and purified from MSC-GcCAHα3, MSC-Saz, MSC-GcCAHα3-M, and MSC-Saz-M and immobilized onto cellulose beads decreased slightly over time, 90% or more thereof was maintained for 40 days (see FIG. 10). From these results, it was concluded that GcCAHα3 immobilized onto cellulose beads maintains activity for a long period of time.

As is apparent from the foregoing description, a recombinant vector according to the present disclosure has the effect of producing a carbonic anhydrase in a plant at low cost and with high efficiency. Specifically, the recombinant vector includes, in the following order, BiP which induces high accumulation in the ER, an M domain which enhances protein expression, CBM3 including a cellulose-binding domain, and HDEL which induces a high accumulation rate in the ER. In addition, the recombinant vector of the present disclosure is a vector produced by sequentially linking, to the above recombinant vector, a translation amplification sequence 5'-UTR, a CaMV 35S promoter, and a HSP terminator. The recombinant vector can be transformed into Agrobacterium and then cultured to thereby produce a carbonic anhydrase in a plant using a vacuum infiltration method at low cost and with high efficiency, and the produced carbonic anhydrase can be effectively separated and purified through cellulose beads. The carbonic anhydrase bound to cellulose beads has an effect of immobilizing the carbonic anhydrase onto the surface of a solid, and the carbonic anhydrase in this state can be used in the hydration reaction of carbon dioxide. The carbonic anhydrase bound to cellulose beads exhibits higher thermal stability than that of a free-form carbonic anhydrase produced using *E. coli.* The carbonic anhydrase bound to cellulose beads has high durability. The carbonic anhydrase bound to cellulose beads can easily be recovered from a reaction solution, and thus has a high reuse rate.

## Claims

1.  A recombinant vector for producing a carbonic anhydrase in a plant, the recombinant vector comprising:

    (i) a small ubiquitin-related modifier (SUMO);
    (ii) a carbonic anhydrase;
    (iii) cellulose-binding module 3 (CBM3); and
    (iv) M domains located before each of the N-terminal of the SUMO and the N-terminal of the CBM3, respectively,

    wherein the gene sequence of each M domain comprises a nucleotide sequence encoding an amino acid sequence of SEQ ID NO: 5, or comprises a nucleotide sequence of SEQ ID NO: 6.

2.  The recombinant vector of claim 1, wherein the carbonic anhydrase is derived from *Gracilariopsis chorda* or *Sulfurihydrogenibium azorense.*

3.  The recombinant vector of claim 2, wherein a gene sequence of the carbonic anhydrase derived from *Gracilariopsis chorda* comprises a nucleotide sequence encoding an amino acid sequence of SEQ ID NO: 1, and a gene sequence of the carbonic anhydrase derived from *Sulfurihydrogenibium azorense* comprises a nucleotide sequence encoding an amino acid sequence of SEQ ID NO: 11.

4.  The recombinant vector of claim 3, wherein the gene sequence of the carbonic anhydrase derived from *Gracilariopsis chorda* comprises a nucleotide sequence of SEQ ID NO: 2, and the gene sequence of the carbonic anhydrase derived from *Sulfurihydrogenibium azorense* comprises a nucleotide sequence of SEQ ID NO: 12.

5.  The recombinant vector of any one of claims 1 to 4, wherein a gene of the SUMO comprises a nucleotide sequence of SEQ ID NO: 7, or wherein a gene of the CBM3 comprises a nucleotide sequence of SEQ ID NO: 8.

6.  The recombinant vector of any one of claims 1 to 5, further comprising any one promoter selected from the group consisting of a 35S promoter derived from a cauliflower mosaic virus, a 19S RNA promoter derived from a cauliflower mosaic virus, an actin protein promoter of a plant, and an ubiquitin protein promoter.

7.  A transformant transformed with the recombinant vector of any one of claims 1 to 6, preferably wherein the transformant is Agrobacterium.

## Patentansprüche

1.  Rekombinanter Vektor zum Herstellen einer Carboanhydrase in einer Pflanze, der rekombinante Vektor umfassend:

    (i) einen kleinen Ubiquitin-bezogenen Modifikator (SUMO);
    (ii) eine Carboanhydrase;

(iii) ein Zellulose-bindendes Modul 3 (CBM3) und

(iv) M-Domänen, die sich vor jedem von dem N-Terminus des SUMO bzw. dem N-Terminus des CBM3 befinden,

wobei die Gensequenz jeder M-Domäne eine Nucleotidsequenz umfasst, die eine Aminosäuresequenz von SEQ ID NO: 5 codiert, oder eine Nukleotidsequenz von SEQ ID NO: 6 umfasst.

2. Rekombinanter Vektor nach Anspruch 1, wobei die Carboanhydrase von *Gracilariopsis chorda* oder *Sulfurihydrogenibium azorense* abgeleitet ist.

3. Rekombinanter Vektor nach Anspruch 2, wobei eine Gensequenz der Carboanhydrase, die von *Gracilariopsis chorda* abgeleitet ist, eine Nukleotidsequenz umfasst, die eine Aminosäuresequenz von SEQ ID NO: 1 codiert, und eine Gensequenz der Carboanhydrase, die von *Sulfurihydrogenibium azorense* abgeleitet ist, eine Nucleotidsequenz umfasst, die eine Aminosäuresequenz von SEQ ID NO: 11 umfasst.

4. Rekombinanter Vektor nach Anspruch 3, wobei die Gensequenz der Carboanhydrase, die von *Gracilariopsis chorda* abgeleitet ist, eine Nukleotidsequenz von SEQ ID NO: 2 umfasst und die Gensequenz der Carboanhydrase, die von *Sulfurihydrogenibium azorense* abgeleitet ist, eine Nukleotidsequenz von SEQ ID NO: 12 umfasst.

5. Rekombinanter Vektor nach einem der Ansprüche 1 bis 4, wobei ein Gen des SUMO eine Nukleotidsequenz von SEQ ID NO: 7 umfasst oder wobei ein Gen des CBM3 eine Nukleotidsequenz von SEQ ID NO: 8 umfasst.

6. Rekombinanter Vektor nach einem der Ansprüche 1 bis 5, weiter umfassend einen Promotor, der ausgewählt ist aus der Gruppe bestehend aus einem 35S-Promotor, der von einem Blumenkohlmosaikvirus abgeleitet ist, einem 19S-RNA-Promotor, der von einem Blumenkohlmosaikvirus abgeleitet ist, einem Actinprotein-Promotor einer Pflanze und einem Ubiquitinprotein-Promotor.

7. Transformante, die mit dem rekombinanten Vektor nach einem der Ansprüche 1 bis 6 transformiert ist, wobei die Transformante vorzugsweise Agrobacterium ist.

## Revendications

1. Vecteur recombinant destiné à produire une anhydrase carbonique chez une plante, le vecteur recombinant comprenant :

(i) un petit modificateur aapparenté à l'ubiquitine (SUMO) ;
(ii) une anhydrase carbonique ;
(iii) un module de liaison à la cellulose 3 (CBM3) ; et
(iv) des domaines M situés avant chacun du N-terminal du SUMO et du N-terminal du CBM3, respectivement,

dans lequel la séquence génique de chaque domaine M comprend une séquence nucléotidique codant pour une séquence d'acides aminés de SEQ ID NO : 5, ou comprend une séquence nucléotidique de SEQ ID NO : 6.

2. Vecteur recombinant selon la revendication 1, dans lequel l'anhydrase carbonique est issue de *Gracilariopsis chorda* ou de *Sulfurihydrogenibium azorense.*

3. Vecteur recombinant selon la revendication 2, dans lequel une séquence génique de l'anhydrase carbonique issue de *Gracilariopsis chorda* comprend une séquence nucléotidique codant pour une séquence d'acides aminés de SEQ ID NO: 1, et une séquence génique de l'anhydrase carbonique issue de *Sulfurihydrogenibium* azorense comprend une séquence nucléotidique codant pour une séquence d'acides aminés de SEQ ID NO : 11.

4. Vecteur recombinant selon la revendication 3, dans lequel la séquence génique de l'anhydrase carbonique issue de *Gracilariopsis chorda* comprend une séquence nucléotidique de SEQ ID NO : 2, et la séquence génique de l'anhydrase carbonique issue de *Sulfurihydrogenibium* azorense comprend une séquence nucléotidique de SEQ ID NO : 12.

5. Vecteur recombinant selon l'une quelconque des revendications 1 à 4, dans lequel un gène du SUMO comprend une séquence nucléotidique de SEQ ID NO : 7, ou dans lequel un gène du CBM3 comprend une séquence nucléotidique de SEQ ID NO : 8.

6. Vecteur recombinant selon l'une quelconque des revendications 1 à 5, comprenant en outre un quelconque promoteur sélectionné parmi le groupe consistant en un promoteur 35S issu d'un virus de la mosaïque du chou-fleur, un promoteur 19S d'ARN issu d'un virus de la mosaïque du chou-fleur, un promoteur de protéine d'actine d'une plante, et un promoteur de protéine d'ubiquitine.

7. Transformant transformé par le vecteur recombinant selon l'une quelconque des revendications 1 à 6, de préférence dans lequel le transformant est une agrobactérie.

FIG. 1

**A** MSC-GcCAHα3-M

**B** SDS-PAGE (MSC-GcCAHα3-M)

**C** Anti-CBM3 antibody (MSC-GcCAHα3-M)

**D** MSC-SazCA-M

**E** SDS-PAGE (MSC-SazCA-M)

**F** Anti-CBM3 antibody (MSC-SazCA-M)

**FIG. 2**

FIG. 3

FIG. 4

FIG. 5

FIG. 6

27

FIG. 7

FIG. 8

FIG. 9

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020180084680
- KR 1020170131207
- WO 2008095057 A
- EP 0120516 B1
- US 4940838 A

**Non-patent literature cited in the description**

- **ISLAM et al.** *Plant Biotechnology Journal,* 2019, vol. 17, 1094-1105
- **HANAHAN, D.** *J. Mol. Biol.,* 1983, vol. 166, 557-580